(19) Europäisches Patentamt
European Patent Office
Office européen des brevets

(11) Veröffentlichungsnummer: **0 308 702 A2**

(12) # EUROPÄISCHE PATENTANMELDUNG

(21) Anmeldenummer: **88114338.2**

(22) Anmeldetag: **02.09.88**

(51) Int. Cl.4: **C07D 209/48 , C07D 207/452 , A01N 37/32 , A01N 39/02 , A01N 37/34 , A01N 41/04 , C07D 405/12 , C07C 93/14 , C07C 93/26 , C07C 79/32 , C07C 79/35**

(30) Priorität: **18.09.87 DE 3731516**

(43) Veröffentlichungstag der Anmeldung: **29.03.89 Patentblatt 89/13**

(84) Benannte Vertragsstaaten: **BE CH DE ES FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Fischer, Reiner, Dr.**
**Rembrandtstrasse 15**
**D-4019 Monheim(DE)**
Erfinder: **Schallner, Otto, Dr.**
**Noldeweg 22**
**D-4019 Monheim(DE)**
Erfinder: **Marhold, Albrecht, Dr.**
**Carl-Duisberg-Strasse 329**
**D-5090 Leverkusen 1(DE)**
Erfinder: **Santel, Hans-Joachim, Dr.**
**Gruenstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Lürssen, Klaus, Dr.**
**August-Kierspel-Strasse 145**
**D-5060 Bergisch-Gladbach 2(DE)**
Erfinder: **Strang, Harry, Dr.**
**Unterdorfstrasse 6A**
**D-4000 Duesseldorf 31(DE)**

(54) **N-Aryl-Stickstoffheterocyclen.**

(57) Die Erfindung betrifft neue N-Aryl-Stickstoffheterocyclen der allgemeinen Formel (I)

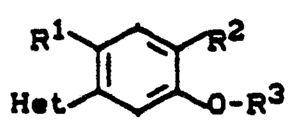

(I)

EP 0 308 702 A2

in welcher
Het für einen Heterocyclus der Formel

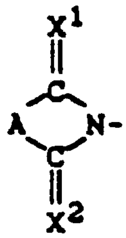 oder 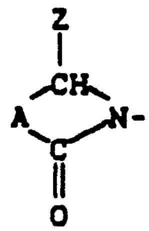 steht,

$R^1$ für Wasserstoff oder Halogen steht,
$R^2$ für Alkyl steht und
$R^3$ für jeweils gegebenenfalls substituiertes Alkyl, Alkenyl, Alkinyl, Cycloalkyl oder Cycloalkenyl steht oder für
einen Rest $-\underset{\underset{O}{\|}}{C}-R^4$
oder einen Rest $-SO_2-R^5$ steht,
A für einen Rest der Formel

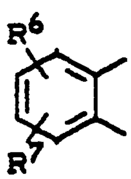 ; 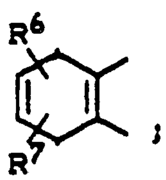 ; 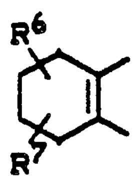 ; 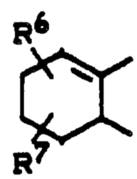 ;

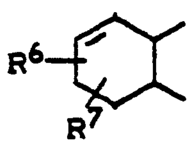 ; 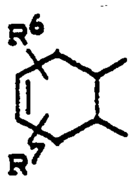 oder 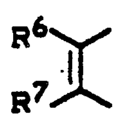 steht und

die restlichen Substituenten die in der Beschreibung angegebene Bedeutung haben, mehrere Verfahren sowie neue Zwischenprodukte zu ihrer Herstellung und ihre Verwendung als Herbizide und Pflanzenwachstumsregulatoren.

## N-Aryl-Stickstoffheterocyclen

Die Erfindung betrifft neue N-Aryl-Stickstoffheterocyclen, mehrere Verfahren zu ihrer Herstellung und ihre Verwendung als Herbizide und Pflanzenwachstumsregulatoren.

Es ist bereits bekannt, daß bestimmte N-Aryl-Stickstoffheterocyclen, wie beispielsweise das N-(2-Fluor-4-chlor-5-propargyloxy-phenyl)-$\Delta^1$-tetrahydrophthalimid oder das N-(2-Fluor-4-chlor-5-methoxycarbonylmethoxy-phenyl)-$\Delta^1$-tetrahydrophthalimid herbizide Eigenschaften besitzen (vgl. EP 83 055 oder EP 61 741). Weiterhin ist das N-[3-(1-Cyanoethoxy)-4-methyl-phenyl]-3,4,5,6-tetrahydrophthalimid aus EP-A 68 822 als Beispiel Nr. 32 bekannt, welches von der Definition der Verbindungen der Formel (I) ausgenommen ist.

Die herbizide Wirksamkeit dieser vorbekannten Verbindungen gegenüber Problemkräutern ist jedoch ebenso wie ihre Verträglichkeit gegenüber bestimmten Kulturpflanzen nicht in allen Anwendungsgebieten völlig zufriedenstellend.

Über eine pflanzenwachstumsregulierende Wirkung der vorbekannten Verbindungen ist bisher nichts bekannt.

Es wurden neue N-Aryl-Stickstoffheterocyclen der allgemeinen Formel (I),

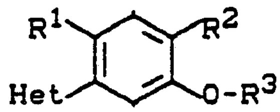

(I)

in welcher

Het für einen Heterocyclus der Formel

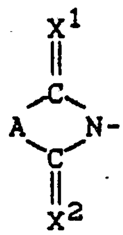

oder

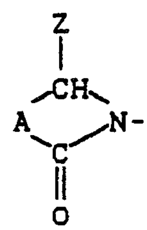

steht,

$R^1$ für Wasserstoff oder Halogen steht,

$R^2$ für Alkyl steht und

$R^3$ für jeweils gegebenenfalls substituiertes Alkyl, Alkenyl, Alkinyl, Cycloalkyl oder Cycloalkenyl steht oder für

einen Rest $-\underset{\overset{\|}{O}}{C}-R^4$

oder einen Rest $-SO_2-R^5$ steht,

A für einen Rest der Formel

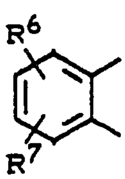

;

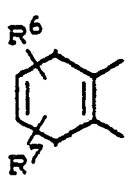

;

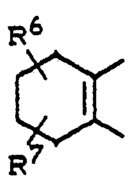

;

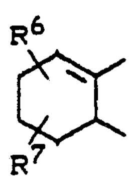

;

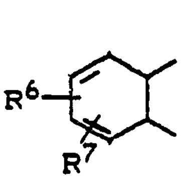

;

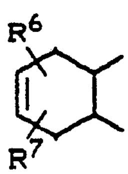

oder

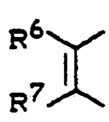

steht,

$X^1$ und $X^2$ jeweils für Sauerstoff oder Schwefel stehen,
Z für Wasserstoff, Hydroxy oder Chlor steht,
$R^4$ für Alkyl, Alkoxy oder Halogenalkyl steht,
$R^5$ für Alkyl, Halogenalkyl oder für gegebenenfalls substituiertes Aryl steht und
$R^6$ und $R^7$ unabhängig voneinander jeweils für Wasserstoff, Halogen, Alkyl oder Halogenalkyl stehen,
wobei jedoch die Verbindung N-[3-(1-Cyanoethoxy)-4-methyl-phenyl]-3,4,5,6-tetrahydrophthalimid ausgenommen ist,
gefunden.

Weiterhin wurde gefunden, daß man die neuen N-Aryl-Stickstoffheterocyclen der allgemeinen Formel (I),

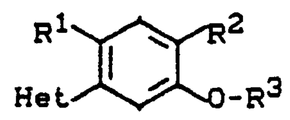

(I)

in welcher
Het für einen Heterocyclus der Formel

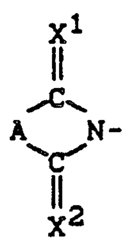

oder

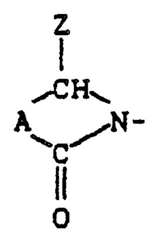

steht,

$R^1$ für Wasserstoff oder Halogen steht,
$R^2$ für Alkyl steht,
$R^3$ für jeweils gegebenenfalls substituiertes Alkyl, Alkenyl, Alkinyl, Cycloalkyl oder Cyloalkenyl steht oder für einen Rest $-\underset{\underset{O}{\|}}{C}-R^4$
oder einen Rest $-SO_2R^5$ steht,
wobei
A für einen Rest der Formel

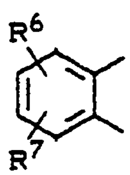

;

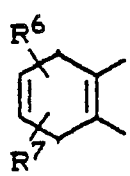

;

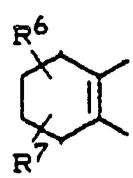

;

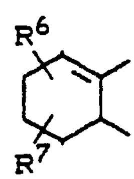

;

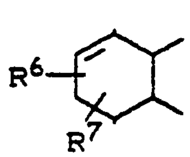

;

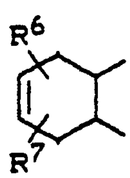

oder

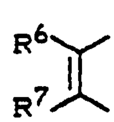

steht,

$X^1$ und $X^2$ jeweils für Sauerstoff oder Schwefel stehen,
Z für Wasserstoff, Hydroxy oder Chlor steht,
$R^4$ für Alkyl, Alkoxy oder Halogenalkyl steht,
$R^5$ für Alkyl, Halogenalkyl oder für gegebenenfalls substituiertes Aryl steht und
$R^6$ und $R^7$ unabhängig voneinander jeweils für Wasserstoff, Halogen, Alkyl oder Halogenalkyl stehen,
wobei jedoch die Verbindung N-[3-(1-Cyanoethoxy)-4-methyl-phenyl]-3,4,5,6-tetrahydrophthalimid ausgenommen ist,

nach einem der im Folgenden beschriebenen Verfahren erhält:

(a) Man erhält N-Arylstickstoffheterocyclen der Formel (Ia),

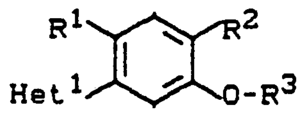

(Ia)

in welcher

Het¹ für einen Rest der Formel

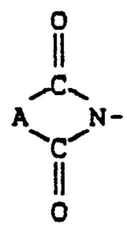

steht und

$R^1$, $R^2$, $R^3$ und A die oben angegebene Bedeutung haben,

wenn man Anhydride der Formel (II),

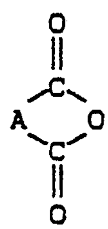

(II)

in welcher

A die oben angegebene Bedeutung hat,

mit Aminen der Formel (III),

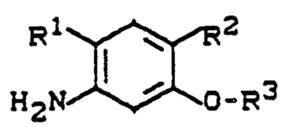

(III)

in welcher

$R^1$, $R^2$ und $R^3$ die oben angegebene Bedeutung haben,

gegebenenfalls in Gegenwart eines Verdünnungsmittels und gegebenenfalls in Gegenwart eines Reaktionshilfsmittels umsetzt;

(b) man erhält N-Aryl-Stickstoffheterocyclen der Formel (Ib),

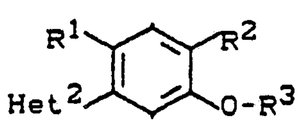

(Ib)

in welcher

Het² für einen Rest der Formel

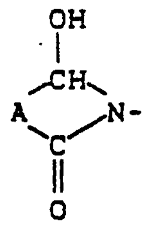 steht und

R', $R^2$, $R^3$ und A die oben angegebene Bedeutung haben,
wenn man N-Arylstickstoffheterocyclen der Formel (Ia),

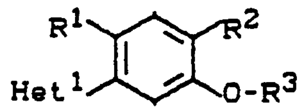 (Ia)

in welcher
$Het^1$ für einen Rest der Formel

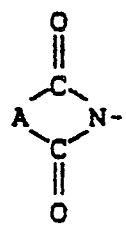 steht und

A, $R^1$, $R^2$ und $R^3$ die oben angegebene Bedeutung haben,
mit einem Reduktionsmittel, gegebenenfalls in Gegenwart eines Verdünnungsmittels umsetzt;
(c) man erhält N-Aryl-Stickstoffheterocyclen der Formel (Ic),

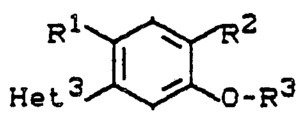 (Ic)

in welcher
$Het^3$ für einen Rest der Formel

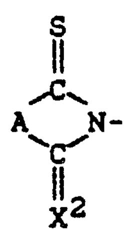 steht und

$X^2$, A, $R^1$, $R^2$ und $R^3$ die oben angegebene Bedeutung haben,
wenn man N-Aryl-Stickstoffheterocyclen der Formel (Ia),

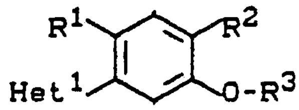

(Ia)

in welcher
Het¹ für einen Rest der Formel

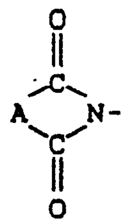

steht und

A, $R^1$, $R^2$ und $R^3$ die oben angegebene Bedeutung haben, mit "Lawessons-Reagenz" der Formel (IV),

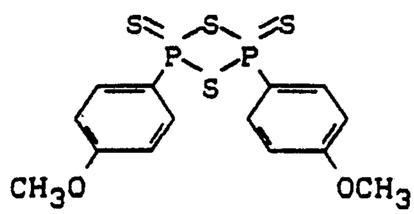

(IV)

gegebenenfalls in Gegenwart eines Verdünnungsmittels umsetzt;

(d) man erhält N-Aryl-Stickstoffheterocyclen der Formel (Id),

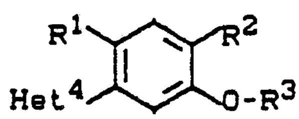

(Id)

in welcher
$Het^4$ für einen Rest der Formel

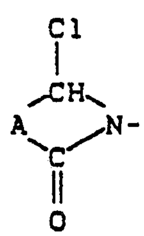

steht und

A, $R^1$, $R^2$ und $R^3$ die oben angegebene Bedeutung haben, wenn man N-Aryl-Stickstoffheterocyclen der Formel (Ib),

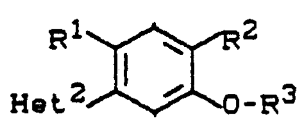

(Ib)

in welcher
Het² für einen Rest der Formel

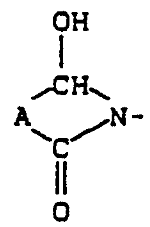

steht und

A, $R^1$, $R^2$ und $R^3$ die oben angegebene Bedeutung haben, mit Thionylchlorid gegebenenfalls in Gegenwart eines Verdünnungsmittels und gegebenenfalls in Gegenwart eines Reaktionshilfsmittels umsetzt;

(e) man erhält N-Aryl-Stickstoffheterocyclen der Formel (Ie),

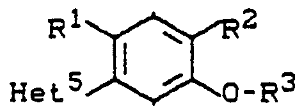

(Ie)

in welcher
Het⁵ für einen Rest der Formel

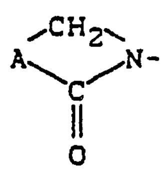

steht und

A, $R^1$, $R^2$ und $R^3$ die oben angegebene Bedeutung haben, wenn man N-Aryl-Stickstoffheterocyclen der Formel (Id),

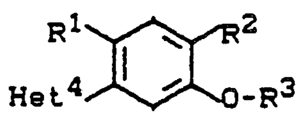

(Id)

in welcher
Het⁴ für einen Rest der Formel

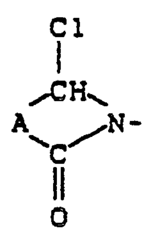

steht und

A, $R^1$, $R^2$ und $R^3$ die oben angegebene Bedeutung haben, mit molekularem Wasserstoff in Gegenwart eines Katalysators sowie gegebenenfalls in Gegenwart eines Verdünnungsmittels und gegebenenfalls in Gegenwart eines Säurebindemittels umsetzt;

(f) man erhält N-Aryl-Stickstoffheterocyclen der Formel (Ia),

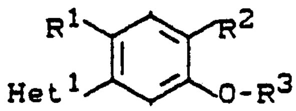

(Ia)

in welcher
$Het^1$ für einen Rest der Formel

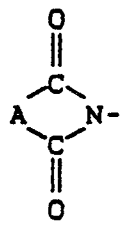 steht und

A, $R^1$, $R^2$ und $R^3$ die oben angegebene Bedeutung haben, alternativ auch, wenn man Phenole der Formel (V),

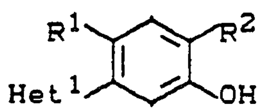

(V)

in welcher
$Het^1$, $R^1$ und $R^2$ die oben angegebene Bedeutung haben,
α) mit Alkylierungsmitteln der Formel (VI),

$R^{3-1}-E^1$ (VI)

in welcher
$R^{3-1}$ für jeweils gegebenenfalls substituiertes Alkyl, Alkenyl, Alkinyl, Cycloalkyl oder Cycloalkenyl steht und
$E^1$ für eine elektronenanziehende Abgangsgruppe steht, oder
(β) mit Acylierungsmitteln der Formel (VII),

$R^4-\underset{\underset{O}{\parallel}}{C}-E^2$ (VII)

in welcher
$R^4$ die oben angegebene Bedeutung hat und
$E^2$ für eine elektronenanziehende Abgangsgruppe steht, oder
(γ) mit Sulfonylierungsmitteln der Formel (VIII),

$R^5-SO_2-E^3$ (VIII)

in welcher
$R^5$ die oben angegebene Bedeutung hat und
$E^3$ für eine elektronenanziehende Abgangsgruppe steht,
jeweils gegebenenfalls in Gegenwart eines Verdünnungsmittels und gegebenenfalls in Gegenwart eines Reaktionshilfsmittels umsetzt.

Schließlich wurde gefunden, daß die neuen N-Aryl-Stickstoffheterocyclen der allgemeinen Formel (I) herbizide und pflanzenwachstumsregulierende Eigenschaften besitzen.

Überraschenderweise zeigen die erfindungsgemäßen N-Aryl-Stickstoffheterocyclen der allgemeinen

Formel (I) neben einer erheblich besseren herbiziden Wirksamkeit gegenüber wichtigen Problemunkräutern gleichzeitig eine deutlich verbesserte Verträglichkeit gegenüber wichtigen Kulturpflanzen im Vergleich zu den aus dem Stand der Technik bekannten N-Aryl-Stickstoffheterocyclen, wie beispielsweise das N-(2-Fluor-4-chlor-5-propargyloxyphenyl)-$\Delta^1$-tetrahydrophthalimid oder das N-(2-Fluor-4-chlor-5-methoxycarbonylmethoxy-phenyl)-$\Delta^1$-tetrahydrophthalimid, welche chemisch und wirkungsmäßig naheliegende Verbindungen sind. Darüber hinaus zeigen die erfindungsgemäßen Verbindungen der Formel (I) unerwarteterweise zusätzlich eine pflanzenwachstumsregulierende Wirkung.

Die erfindungsgemäßen N-Aryl-Stickstoffheterocyclen sind durch die Formel (I) allgemein definiert.

Bevorzugt sind Verbindungen der Formel (I), bei welchen

Het für einen Heterocyclus der Formel

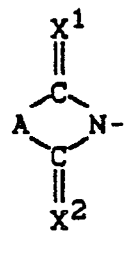 oder 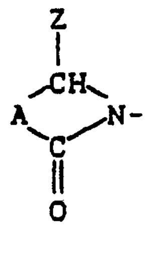 steht,

$R^1$ für Wasserstoff, Fluor, Chlor oder Brom steht,

$R^2$ für geradkettiges oder verzweigtes Alkyl mit 1 bis 4 Kohlenstoffatomen steht,

$R^3$ für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen steht, außerdem für jeweils geradkettiges oder verzweigtes Cyanalkyl, Alkoxyalkyl, Alkylthioalkyl, Halogenalkoxyalkyl, Alkoxyalkoxyalkyl, Alkoxyalkoxyalkoxyalkyl, Bis-(alkoxy)alkyl, Bis(alkylthio)alkyl, Alkylalkoxycarbonylalkyl mit jeweils 1 bis 8 Kohlenstoffatomen in den einzelnen Alkylteilen und gegebenenfalls 1 bis 9 gleichen oder verschiedenen Halogenatomen steht, ferner für jeweils gegebenenfalls einfach oder mehrfach, gleich oder verschieden substituiertes Cycloalkyl, Cycloalkenyl, Cycloalkyloxycarbonylalkyl oder Cycloalkylalkyl mit jeweils 3 bis 7 Kohlenstoffatomen im Cycloalkylteil bzw. im Cycloalkenylteil und gegebenenfalls 1 bis 4 Kohlenstoffatomen im geradkettigen oder verzweigten Alkylteil steht, wobei als Substituenten jeweils infrage kommen: Halogen sowie jeweils geradkettiges oder verzweigtes Alkyl oder Alkoxy mit jeweils 1 bis 4 Kohlenstoffatomen, $R^3$ außerdem für jeweils gegebenenfalls einfach oder mehrfach gleich oder verschieden substituiertes Oxiranylalkyl, Oxetanylalkyl, Tetrahydrofuranylalkyl oder Tetrahydropyranylalkyl mit jeweils 1 bis 4 Kohlenstoffatomen im geradkettigen oder verzweigten Alkylteil steht, wobei als Substituenten jeweils infrage kommen: Halogen, sowie jeweils geradkettiges oder verzweigtes Alkyl oder Alkoxy mit jeweils 1 bis 4 Kohlenstoffatomen; $R^3$ weiterhin für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Substituenten infrage kommen:

Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen; oder $R^3$ ferner für einen Rest $-\underset{\underset{O}{\|}}{C}-R^4$

oder für einen Rest $-SO_2-R^5$ steht,

A für einen Rest der Formel

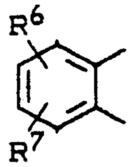 ; 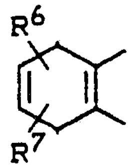 ; 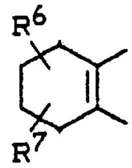 ; 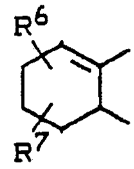 ;

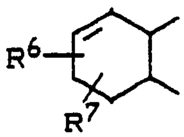 ; 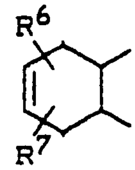 oder 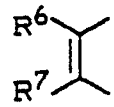 steht,

$X^1$ und $X^2$ jeweils für Sauerstoff oder Schwefel stehen,

Z für Wasserstoff, Hydroxy oder Chlor steht,

$R^4$ für jeweils geradkettiges oder verzweigtes Alkyl, Alkoxy oder Halogenalkyl mit jeweils 1 bis 6 Kohlenstoffatomen und im Fall des Halogenalkyl mit 1 bis 13 gleichen oder verschiedenen Halogenatomen steht,

$R^5$ für jeweils geradkettiges oder verzweigtes Alkyl oder Halogenalkyl mit jeweils 1 bis 6 Kohlenstoffatomen und im Fall des Halogenalkyl mit 1 bis 13 gleichen oder verschiedenen Halogenatomen steht und außerdem für gegebenenfalls einfach oder mehrfach, gleich oder verschieden substituiertes Phenyl steht, wobei als Substituenten infrage kommen:

Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen und

$R^6$ und $R^7$ unabhängig voneinander jeweils für Wasserstoff, Fluor, Chlor, Brom oder für jeweils geradkettiges oder verzweigtes Alkyl oder Halogenalkyl mit jeweils 1 bis 3 Kohlenstoffatomen und im Fall des Halogenalkyl mit .1 bis 7 gleichen oder verschiedenen Halogenatomen, insbesondere Fluor, Chlor oder Brom stehen,

wobei jedoch die Verbindung N-[3-(1-Cyanoethoxy)-4-methyl-phenyl]-3,4,5,6-tetrahydrophthalimid ausgenommen ist.

Besonders bevorzugt sind Verbindungen der Formel (I), bei welchen

Het für einen Heterocyclus der Formel

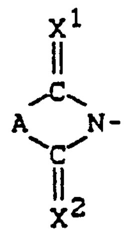 oder 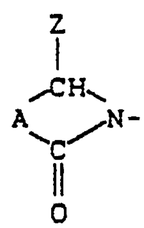 steht,

$R^1$ für Wasserstoff, Fluor oder Chlor steht,

$R^2$ für Methyl oder Ethyl steht,

$R^3$ für Methyl, Ethyl, n- oder i-Propyl, n-, i-, s-oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, insbesondere Fluor, Chlor oder brom steht, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis sechsfach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclobutenyl, Cyclopentenyl, Cyclohexenyl, Cyclopropyl, Cyclobutyl, Cyclopentyl oder Cyclohexyl steht, außerdem für jeweils gegebenenfalls ein- bis

dreifach gleich oder verschieden durch Fluor, Chlor, Methyl oder Ethyl substituiertes Oxiranylmethyl, Oxetanylmethyl, Tetrahydrofuranylmethyl oder Tetrahydropyranylmethyl steht oder für jeweils gegebenenfalls ein- bis dreifach, gleich oder verschieden substituiertes Benzyl oder Phenylethyl steht, wobei als Substituenten jeweils infrage kommen:
Fluor, Chlor, Brom, Cyano, Nitro, Methyl, Ethyl, n- oder i-Propyl, n-, i-, s- oder t-butyl, Methoxy, Ethoxy, n- oder i-Propoxy, Methylthio, Ethylthio, Methoxycarbonyl, Ethoxycarbonyl, Trifluormethyl, Trifluormethoxy oder Triflormethylthio; $R^3$ ferner für einen Rest $-\underset{O}{\underset{\|}{C}}-R^4$

oder für einen Rest $-SO_2R^5$ steht,
A für einen Rest der Formel

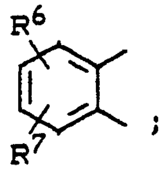 ; 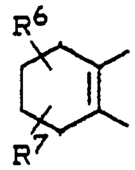 oder 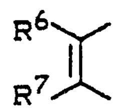 steht,

$X^1$ and $X^2$ jeweils für Sauerstoff oder Schwefel stehen,
Z für Wasserstoff, Hydroxy oder Chlor steht,
$R^4$ für jeweils geradkettiges oder verzweigtes Alkyl, Alkoxy oder Halogenalkyl mit jeweils 1 bis 3 Kohlenstoffatomen und im Fall des Halogenalkyl mit 1 bis 7 gleichen oder verschiedenen Halogenatomen steht,
$R^5$ für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen steht oder für gegebenenfalls ein- bis dreifach, gleich oder verschieden substituiertes Phenyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio und
$R^6$ und $R^7$ unabhängig voneinander jeweils für Wasserstoff, Chlor, Methyl oder Trifluormethyl stehen,
wobei jedoch die Verbindung N-[3-(1-Cyanoethoxy)-4-methyl-phenyl]-3,4,5,6-tetrahydrophthalimid ausgenommen ist.

Ganz besonders bevorzugt sind Verbindungen der Formel (I), bei welchen
Het für einen Heterocyclus der Formel

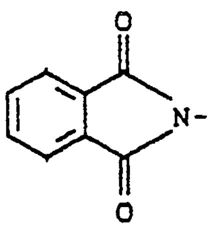 ; 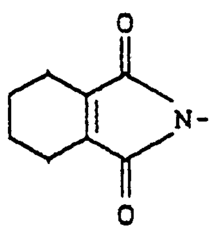 oder 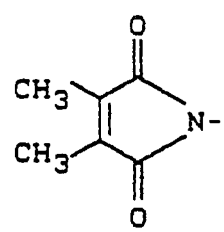

steht,
$R^1$ für Wasserstoff oder Fluor steht,
$R^2$ für Methyl steht,
$R^3$ für Methyl, Ethyl, n- oder i-Propyl, n-, i-, s-oder t-Butyl, für Allyl, Propargyl, für jeweils geradkettiges oder verzweigtes Pentyl, Hexyl, Butenyl, pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor, Chlor oder Brom steht, für jeweils geradkettiges oder verzweigtes Cyanalkyl, Alkoxyalkyl, Alkoxyalkoxyalkyl oder Alkoxycarbonylalkyl mit jeweils 1 bis 5 Kohlenstoffatomen in den einzelnen Alkylteilen steht, außerdem für jeweils gegebenenfalls ein- bis sechsfach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclobutenyl, Cyclopentenyl, Cyclohexenyl, Cyclobutyl, Cyclopropyl, Cyclopentyl oder Cyclohexyl steht, außerdem für gegebenenfalls einfach durch Methyl oder Ethyl substituiertes Oxetanylmethyl, Tetrahydrofuranylmethyl oder Tetrahydropyranylmethyl steht oder für jeweils gegebenenfalls ein-bis dreifach gleich oder verschieden substituiertes Benzyl oder Phenylethyl steht, wobei als Substituenten jeweils in Frage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio; $R^3$ ferner für einen Rest - 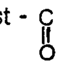 -$R^4$

oder für einen Rest -$SO_2$-$R^5$ steht, wobei
$R^4$ für jeweils geradkettiges oder verzweigtes Alkyl, Alkoxy oder Halogenalkyl mit jeweils 1 bis 3 Kohlenstoffatomen und im Fall des Halogenalkyl mit 1 bis 7 gleichen oder verschiedenen Halogenatomen steht,
$R^5$ für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen steht oder für gegebenenfalls ein- bis dreifach gleich oder verschieden substituiertes Phenyl steht, wobei als Substituenten infrage kommen:
Fluor, Chlor, Brom, Cyano, Nitro, Methyl, Ethyl, n- oder i-Propyl, n-, i-, s- oder t-Butyl, methoxy, Ethoxy, n- oder i-Propoxy, Methylthio, Ethylthio, Methoxycarbonyl, Ethoxycarbonyl, Trifluormethyl, Trifluormethoxy oder Trifluormethylthio, wobei jedoch die Verbindung N-[3-(1-Cyanoethoxy)-4-methyl-phenyl]-3,4,5,6-tetrahydrophthalimid ausgenommen ist.

Im einzelnen seien außer den bei den Herstellungsbeispielen genannten Verbindungen die folgenden N-Aryl-Stickstoffheterocyclen der allgemeinen Formel (I) genannt:

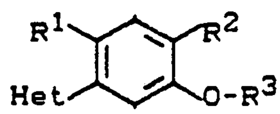

(I)

| Het | $R^1$ | $R^2$ | $R^3$ |
|---|---|---|---|
| 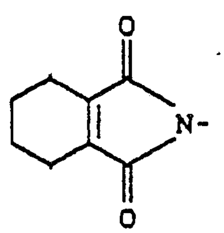 | F | $CH_3$ | $-CH_2-CN$ |
| 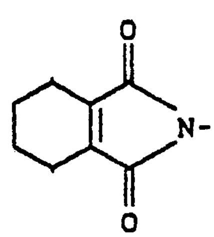 | F | $CH_3$ | 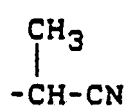 |

| Het | $R^1$ | $R^2$ | $R^3$ |
|---|---|---|---|
| 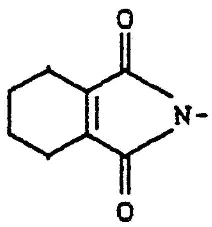 | F | $CH_3$ | 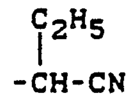 |
| 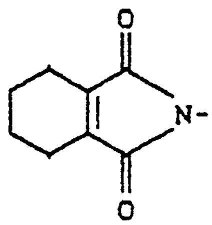 | F | $CH_3$ | 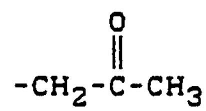 |
| 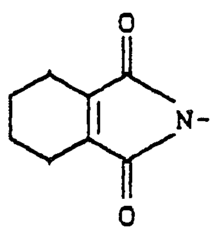 | F | $CH_3$ | 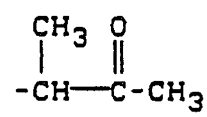 |
| 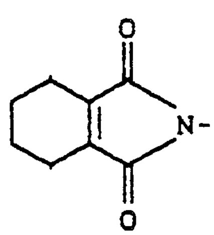 | F | $CH_3$ | 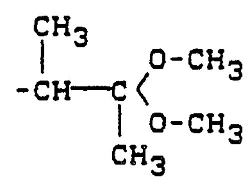 |
| 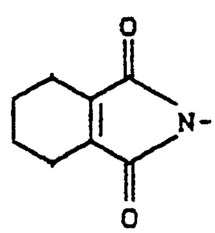 | F | $CH_3$ | 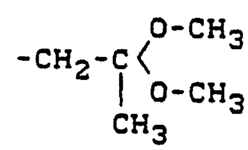 |

| Het | $R^1$ | $R^2$ | $R^3$ |
|---|---|---|---|
| 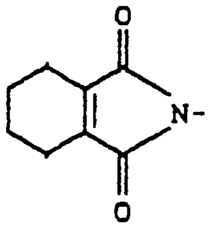 | F | $CH_3$ | $-CF_2-CHF-Cl$ |
| 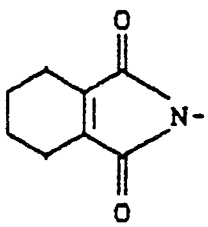 | F | $CH_3$ | $-CF_2-CHF-CF_3$ |
| 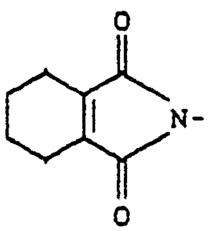 | F | $CH_3$ | 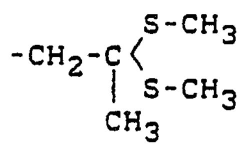 |
| 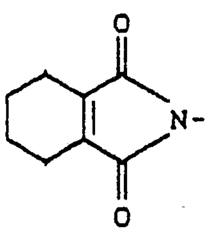 | F | $CH_3$ | $-CH(CH_2F)_2$ |
| 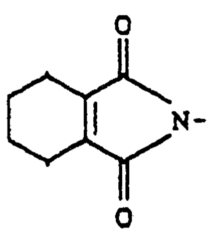 | F | $CH_3$ | $-CH_2-CH_2-O-CH_2-CF_3$ |

| Het | $R^1$ | $R^2$ | $R^3$ |
|---|---|---|---|
| 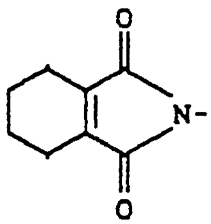 | F | $CH_3$ | $CH_3$<br>\|<br>$-CH-CH_2-O-CH_2-CF_3$ |
| 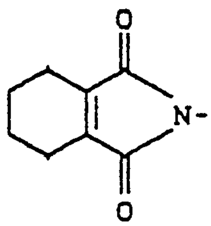 | F | $CH_3$ | Cl<br>\|<br>$-CH_2-C=CH_2$ |
| 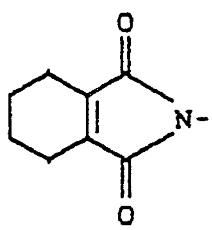 | F | $CH_3$ | $-CH_2-CH_2F$ |
| 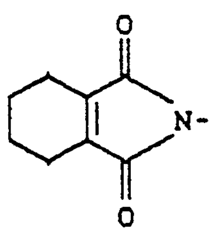 | F | $CH_3$ | $-CF_2-CHF-CF_3$ |
| 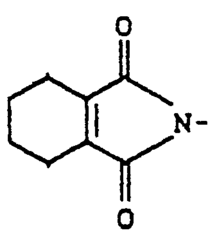 | F | $CH_3$ | $-CF_2-CHF_2$ |

| Het | $R^1$ | $R^2$ | $R^3$ |
|---|---|---|---|
| 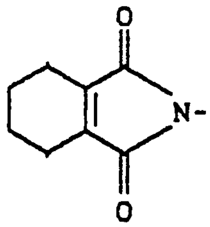 | F | $CH_3$ | $-CF_2-CHClF$ |
| 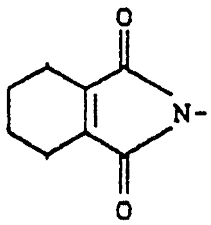 | F | $CH_3$ | $-CF_3$ |
| 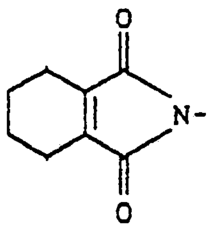 | F | $CH_3$ | $-CClF_2$ |
| 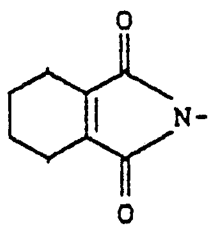 | F | $CH_3$ | $-CH_2-CH=CH-Cl$ |
| 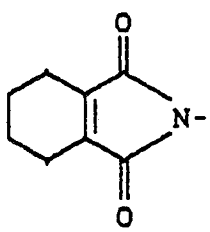 | F | $CH_3$ | $-CH_2-CH_2-Cl$ |

| Het | $R^1$ | $R^2$ | $R^3$ |
| --- | --- | --- | --- |
| 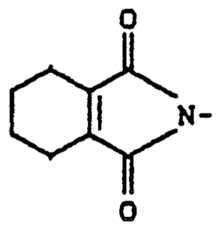 | F | $CH_3$ | $-CH_2-CH_2-O-CH_3$ |
| 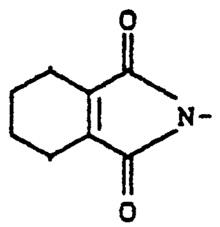 | F | $CH_3$ | $-CH_2-CH_2-O-C_2H_5$ |
| 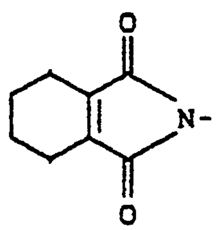 | F | $CH_3$ | $-CH_2-CH_2-O-CH(CH_3)_2$ |
| 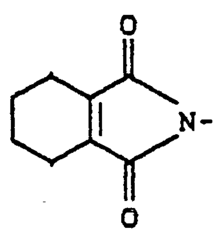 | F | $CH_3$ | $-CH_2-CH_2-O-(CH_2)_2-CH_3$ |
| 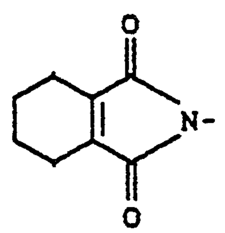 | F | $CH_3$ | $-CH_2-CH_2-O-(CH_2)_3-CH_3$ |

| Het | $R^1$ | $R^2$ | $R^3$ |
|---|---|---|---|
| 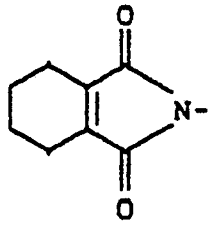 | F | $CH_3$ | $-(CH_2-CH_2-O)_2-CH_3$ |
| 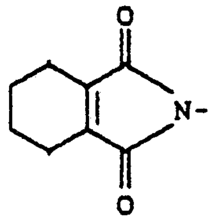 | F | $CH_3$ | $-(CH_2-CH_2-O)_2-CH(CH_3)_2$ |
| 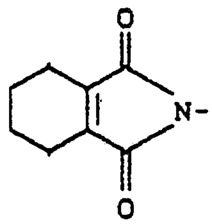 | F | $CH_3$ | $-(CH_2-CH_2-O)_2-(CH_2)_2-CH_3$ |
| 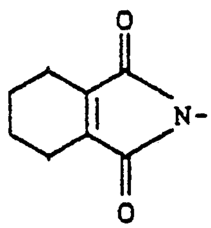 | F | $CH_3$ | $-(CH_2-CH_2-O)_2-(CH_2)_3-CH_3$ |
| 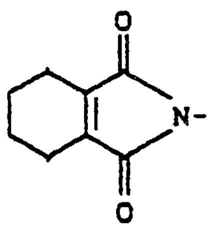 | F | $CH_3$ | $-(CH_2-CH_2-O)_3-CH_3$ |

| Het | $R^1$ | $R^2$ | $R^3$ |
|---|---|---|---|
| 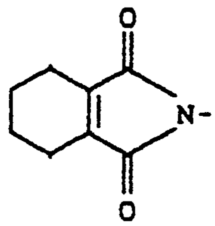 | F | $CH_3$ | $-(CH_2-CH_2-O)_3-C_2H_5$ |
| 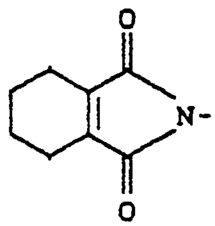 | F | $CH_3$ | $-(CH_2-CH_2-O)_3-(CH_2)_3CH_3$ |
| 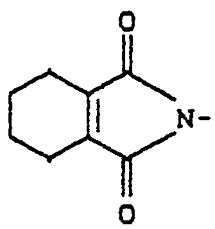 | F | $CH_3$ | $CH_3$<br>\|<br>$-CH-CH_2-O-CH_3$ |
| 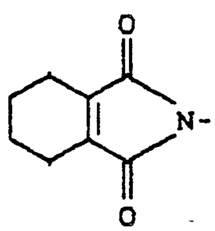 | F | $CH_3$ | $CH_3$<br>\|<br>$-CH-CH_2-O-C_2H_5$ |
| 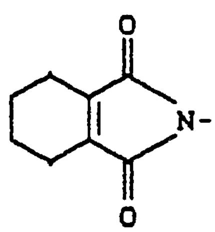 | F | $CH_3$ | $CH_3$<br>\|<br>$-CH-CH_2-O-CH(CH_3)_2$ |

| Het | $R^1$ | $R^2$ | $R^3$ |
|---|---|---|---|
| 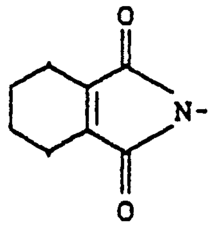 | F | $CH_3$ | 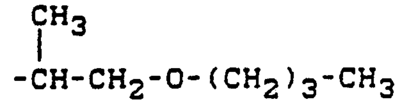 |
| 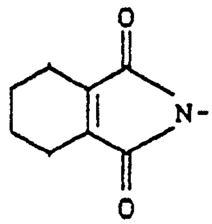 | F | $CH_3$ | 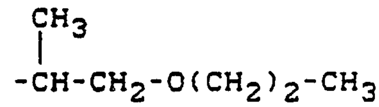 |
| 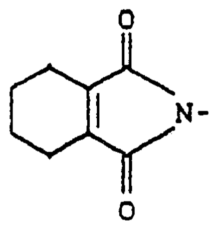 | F | $CH_3$ | 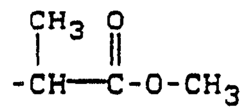 |
| 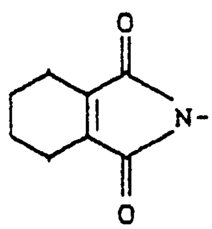 | F | $CH_3$ | 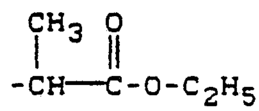 |
| 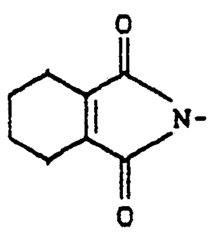 | F | $CH_3$ | 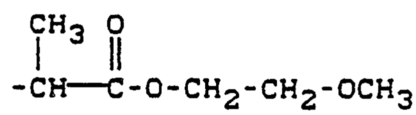 |

| Het | $R^1$ | $R^2$ | $R^3$ |
| --- | --- | --- | --- |
| 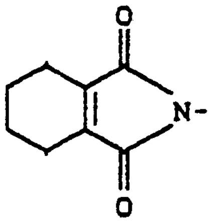 | F | $CH_3$ | 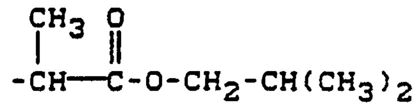 |
| 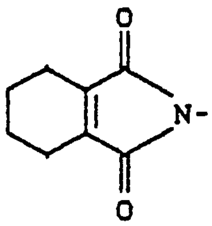 | F | $CH_3$ | 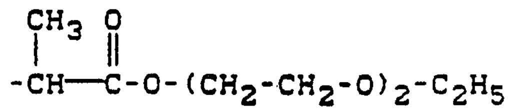 |
| 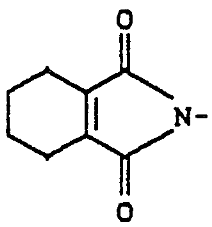 | F | $CH_3$ | 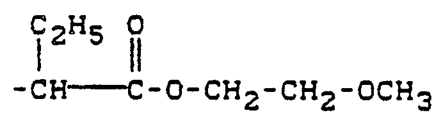 |
| 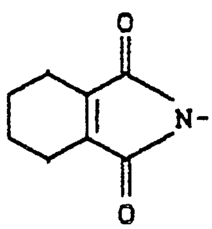 | F | $C_2H_5$ | $-CH_2-C{\equiv}CH$ |
| 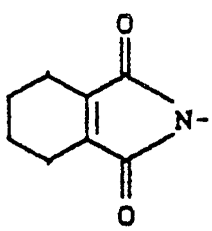 | F | $C_2H_5$ | $-CH_2-CH_2-OC_2H_5$ |

| Het | $R^1$ | $R^2$ | $R^3$ |
|---|---|---|---|
| 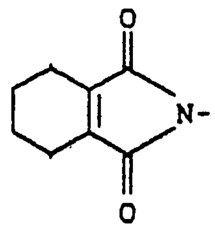 | F | $C_2H_5$ | $-(CH_2-CH_2-O)_2-CH_3$ |
| 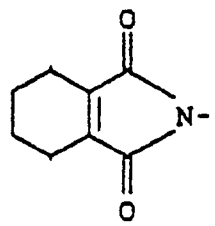 | F | $C_2H_5$ | $-CH_2-CH=CH_2$ |
| 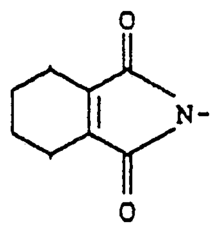 | F | $C_2H_5$ | $-CF_3$ |
| 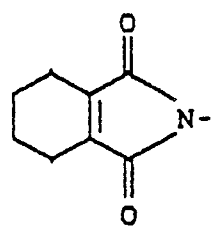 | F | $C_2H_5$ | $-CHF_2$ |
| 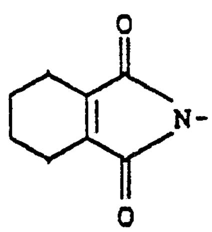 | F | $C_2H_5$ | 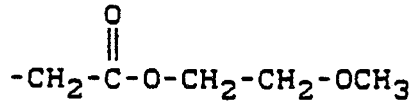 |

| Het | $R^1$ | $R^2$ | $R^3$ |
|---|---|---|---|
| 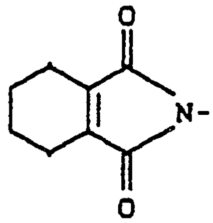 | F | $C_2H_5$ | $CH_3$<br>$-CH-COOC_2H_5$ |
| 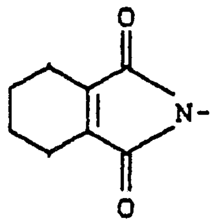 | F | $C_2H_5$ | $-CF_2-CHF-CHCl$ |
| 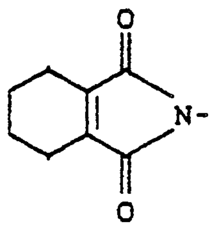 | F | $C_2H_5$ | $-CH(CH_3)_2$ |
| 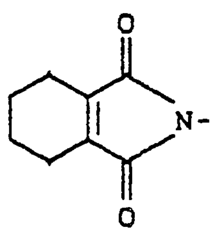 | F | $C_2H_5$ | $-CH_2-C\equiv C-CH_3$ |
| 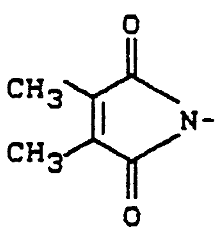 | F | $CH_3$ | $CH_3$<br>$-CH-CH_2-O-CH_3$ |

| Het | $R^1$ | $R^2$ | $R^3$ |
|---|---|---|---|
| 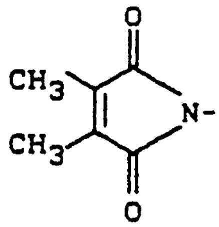 | F | $CH_3$ | 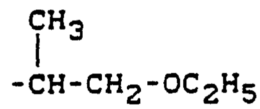 |
| 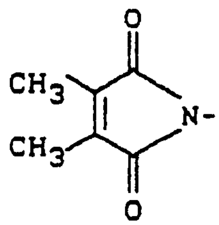 | F | $CH_3$ | 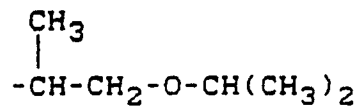 |
| 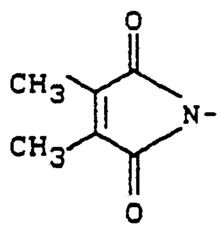 | F | $CH_3$ | 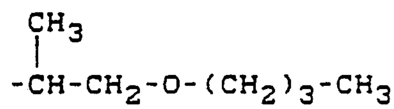 |
| 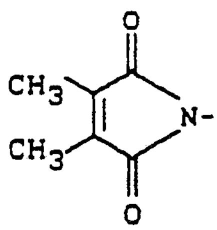 | F | $CH_3$ | 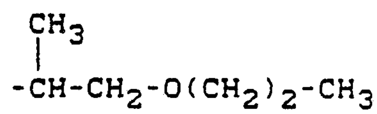 |
| 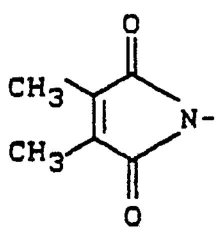 | F | $CH_3$ | 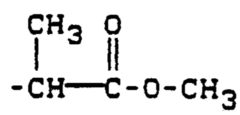 |

| Het | $R^1$ | $R^2$ | $R^3$ |
|---|---|---|---|
| 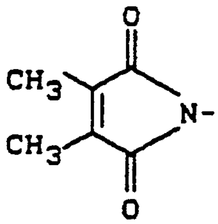 | F | $CH_3$ | 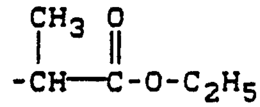 |
| 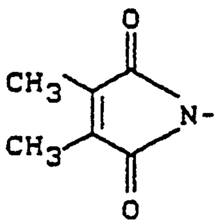 | F | $CH_3$ | 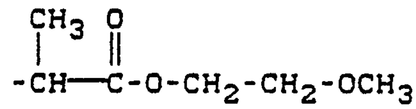 |
| 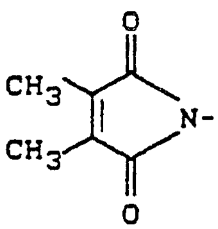 | F | $CH_3$ | 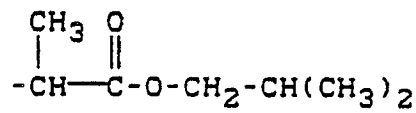 |
| 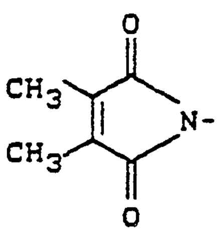 | F | $CH_3$ | 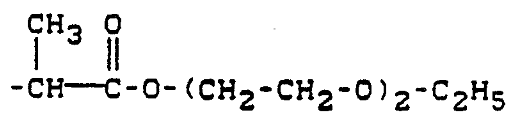 |
| 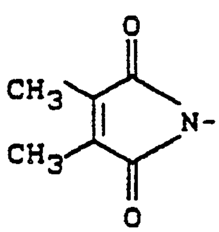 | F | $CH_3$ | 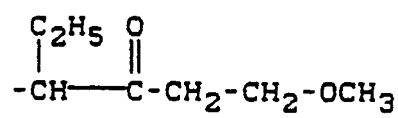 |

| Het | $R^1$ | $R^2$ | $R^3$ |
|---|---|---|---|
| 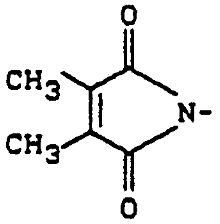 | F | $CH_3$ | $-CH_2-CH_2-O-CH_3$ |
| 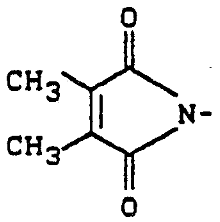 | F | $CH_3$ | $-CH_2-CH_2-O-C_2H_5$ |
| 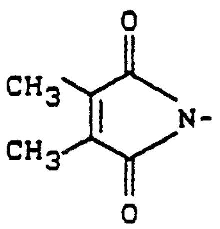 | F | $CH_3$ | $-CH_2-CH_2-O-CH(CH_3)_2$ |
| 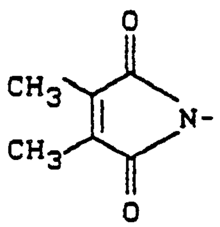 | F | $CH_3$ | $-CH_2-CH_2-O-(CH_2)_2-CH_3$ |
| 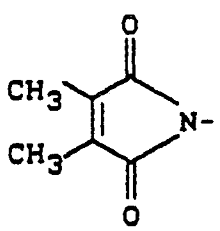 | F | $CH_3$ | $-CH_2-CH_2-O-(CH_2)_3-CH_3$ |

| Het | $R^1$ | $R^2$ | $R^3$ |
|---|---|---|---|
| 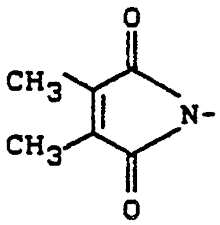 | F | $CH_3$ | $-(CH_2-CH_2-O)_2-CH_3$ |
| 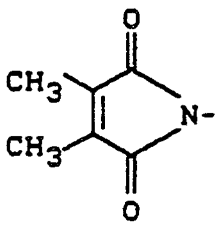 | F | $CH_3$ | $-(CH_2-CH_2-O)_2-C_2H_5$ |
| 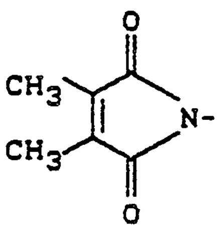 | F | $CH_3$ | $-(CH_2-CH_2-O)_2-CH(CH_3)_2$ |
| 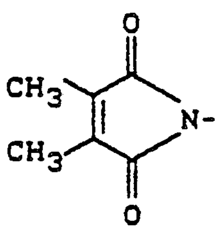 | F | $CH_3$ | $-(CH_2-CH_2-O)_2-(CH_2)_2-CH_3$ |
| 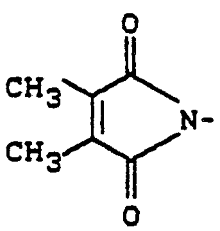 | F | $CH_3$ | $-(CH_2-CH_2-O)_2-(CH_2)_3-CH_3$ |

| Het | $R^1$ | $R^2$ | $R^3$ |
|---|---|---|---|
| 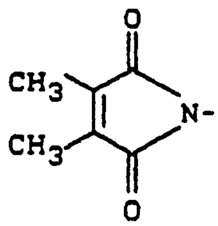 | F | $CH_3$ | $-(CH_2-CH_2-O)_3-CH_3$ |
| 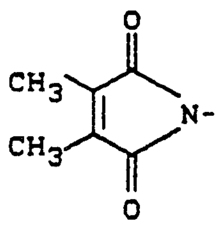 | F | $CH_3$ | $-(CH_2-CH_2-O)_3-C_2H_5$ |
| 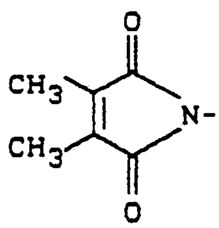 | F | $CH_3$ | $-(CH_2-CH_2-O)_3-(CH_2)_3-CH_3$ |
| 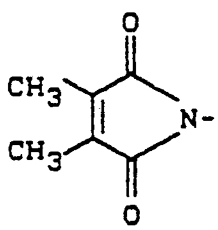 | F | $CH_3$ | $-CH(CH_2F)_2$ |
| 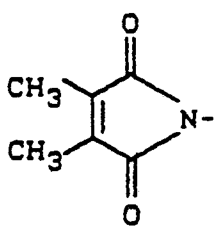 | F | $CH_3$ | $-CH_2-CH_2-O-CH_2-CH(CH_3)_2$ |

| Het | $R^1$ | $R^2$ | $R^3$ |
|---|---|---|---|
| 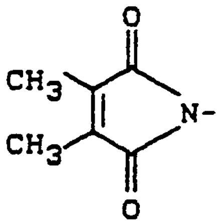 | F | $CH_3$ | 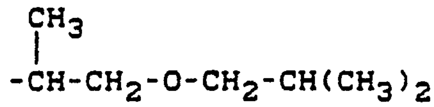 |
| 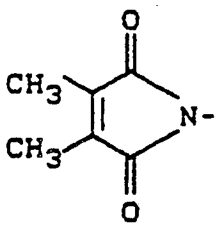 | F | $CH_3$ | 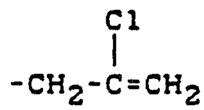 |
| 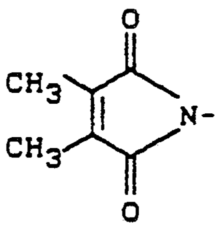 | F | $CH_3$ | $-CH_2-CH_2F$ |
| 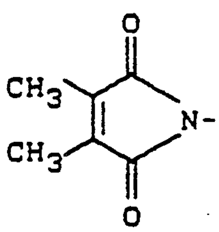 | F | $CH_3$ | $-CF_2-CHF-CF_3$ |
| 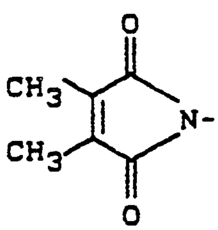 | F | $CH_3$ | $-CF_2-CHF_2$ |

| Het | $R^1$ | $R^2$ | $R^3$ |
|---|---|---|---|
| 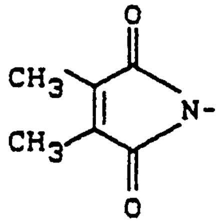 | F | $CH_3$ | $-CF_2-CHClF$ |
| 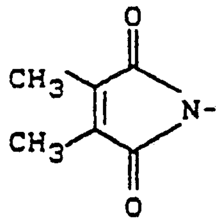 | F | $CH_3$ | $-CF_3$ |
| 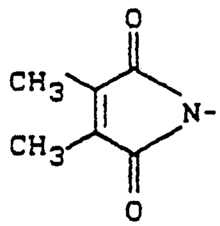 | F | $CH_3$ | $-CClF_2$ |
| 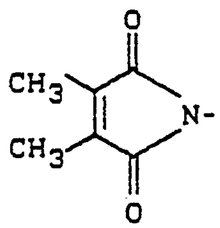 | F | $CH_3$ | $-CH_2-CH=CH-Cl$ |
| 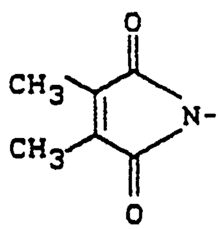 | F | $CH_3$ | $-CH_2-CH_2-Cl$ |

| Het | $R^1$ | $R^2$ | $R^3$ |
|---|---|---|---|
| 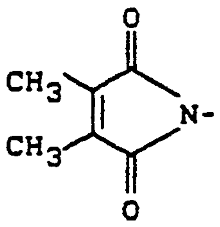 | F | $CH_3$ | $-CH_2-CN$ |
| 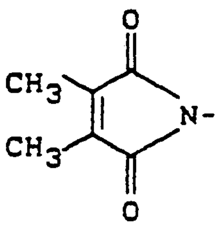 | F | $CH_3$ | 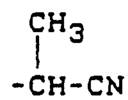 |
| 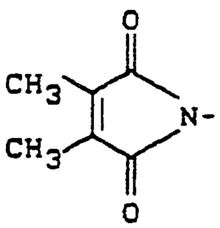 | F | $CH_3$ | 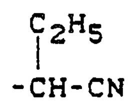 |
| 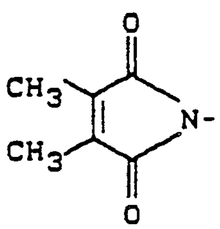 | F | $CH_3$ | 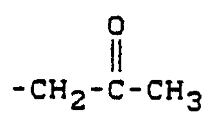 |
| 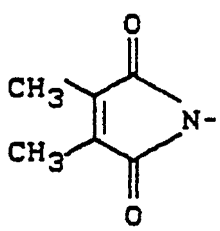 | F | $CH_3$ | 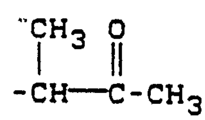 |

| Het | $R^1$ | $R^2$ | $R^3$ |
|---|---|---|---|
| 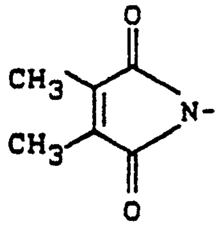 | F | $CH_3$ | 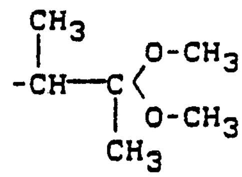 |
| 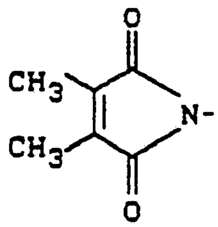 | F | $CH_3$ | 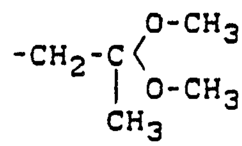 |
| 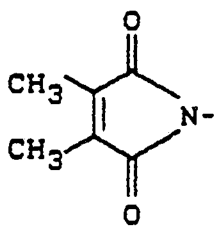 | F | $CH_3$ | $-CF_2-CHFCl$ |
| 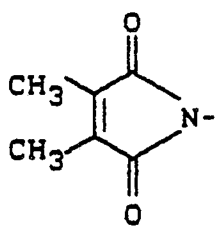 | F | $CH_3$ | $-CF_2-CHF-CF_3$ |
| 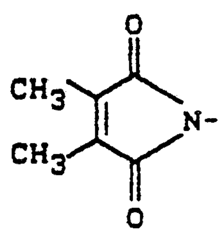 | F | $CH_3$ | 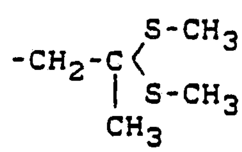 |

| Het | $R^1$ | $R^2$ | $R^3$ |
|---|---|---|---|
| 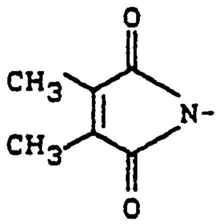 | F | $CH_3$ | $-CH_2-C\equiv CH$ |
| 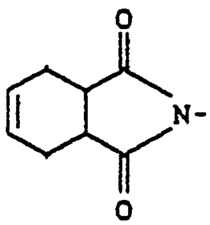 | F | $CH_3$ | $-CH_2-C\equiv CH$ |
| 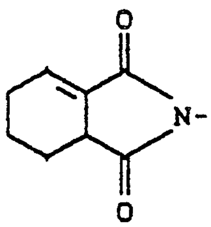 | F | $CH_3$ | $-CH_2-C\equiv CH$ |
| 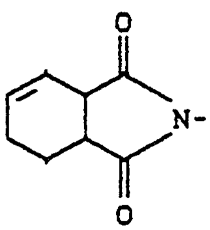 | F | $CH_3$ | $-CH_2-C\equiv CH$ |
| 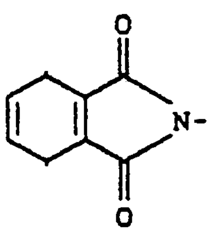 | F | $CH_3$ | $-CH_2-C\equiv CH$ |

| Het | $R^1$ | $R^2$ | $R^3$ |
| --- | --- | --- | --- |
| 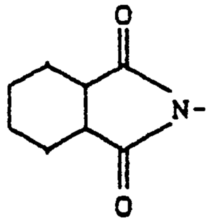 | F | $CH_3$ | $-CH_2-C\equiv C-CH_3$ |
| 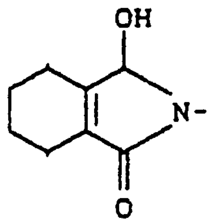 | F | $CH_3$ | $-CH_2-C\equiv CH$ |
| 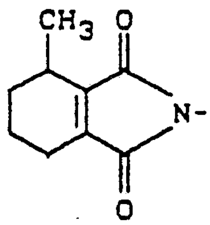 | F | $CH_3$ | $-CH_2-CH=CH-CH_3$ |
| 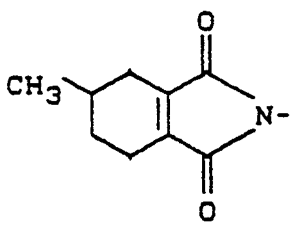 | H | $CH_3$ | $-CH_2-C\equiv CH$ |
| 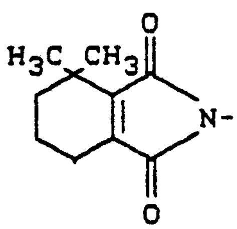 | F | $CH_3$ | $-CH_2-C\equiv CH$ |

| Het | $R^1$ | $R^2$ | $R^3$ |
|---|---|---|---|
| 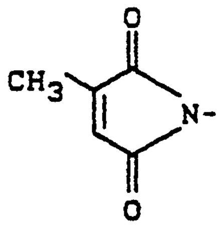 | F | $CH_3$ | $-CH_2-C{\equiv}CH$ |
| 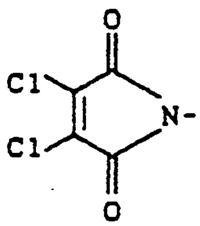 | F | $CH_3$ | $-CH_2-C{\equiv}CH$ |
| 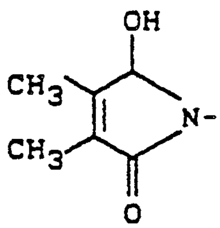 | F | $CH_3$ | $-CH_2-C{\equiv}CH$ |
| 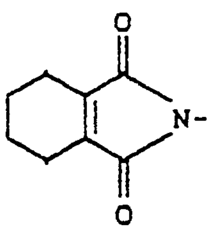 | F | $-CH(CH_3)_2$ | $-CH_2-C{\equiv}CH$ |
| 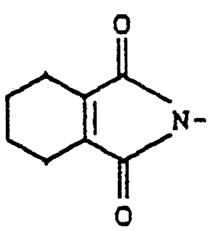 | F | $-CH(CH_3)_2$ | $-CH_2-CH_2-OC_2H_5$ |

| Het | $R^1$ | $R^2$ | $R^3$ |
|---|---|---|---|
| 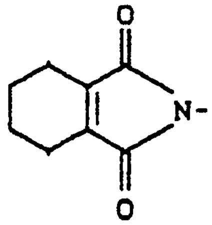 | F | $-CH(CH_3)_2$ | $-(CH_2-CH_2-O)_2-CH_3$ |
| 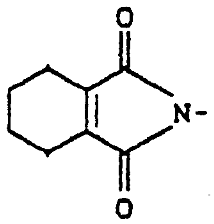 | F | $-CH(CH_3)_2$ | $-CH_2-CH=CH_2$ |
| 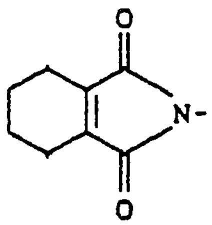 | F | $-CH(CH_3)_2$ | $-CF_3$ |
| 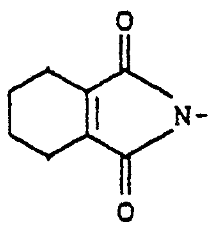 | F | $-CH(CH_3)_2$ | $-CHF_2$ |
| 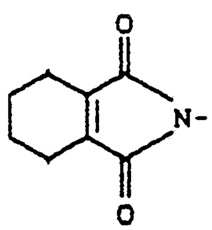 | F | $-CH(CH_3)_2$ | $-CH_2-COOCH_2-CH_2-OCH_3$ |

| Het | $R^1$ | $R^2$ | $R^3$ |
|---|---|---|---|
| 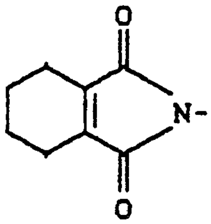 | F | $-CH(CH_3)_2$ | $CH_3$<br>$-CH-COOC_2H_5$ |
| 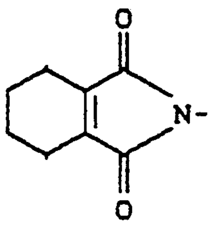 | F | $-CH(CH_3)_2$ | $-CF_2-CHF-CHClF$ |
| 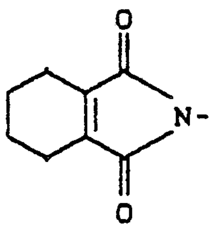 | F | $-CH(CH_3)_2$ | $-CH(CH_3)_2$ |
| 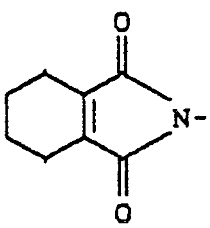 | F | $-CH(CH_3)_2$ | $-CH-C{\equiv}C-CH_3$ |
| 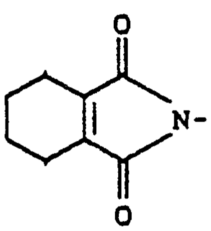 | F | $-(CH_2)_2-CH_3$ | $-CH_2-C{\equiv}CH$ |

| Het | $R^1$ | $R^2$ | $R^3$ |
|---|---|---|---|
| 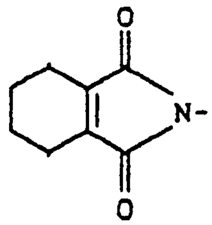 | F | $-(CH_2)_2-CH_3$ | $-CH_2-CH_2-OC_2H_5$ |
| 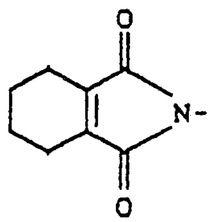 | F | $-(CH_2)_2-CH_3$ | $-(CH_2-CH_2-O)_2-CH_3$ |
| 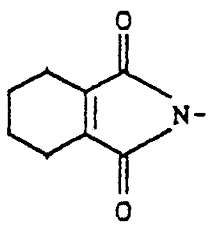 | F | $-(CH_2)_2-CH_3$ | $-CH_2-CH=CH_2$ |
| 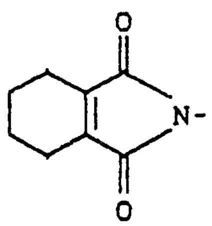 | F | $-(CH_2)_2-CH_3$ | $-CF_3$ |
| 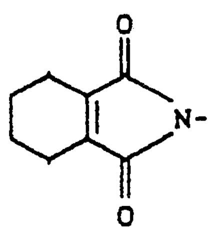 | F | $-(CH_2)_2-CH_3$ | $-CHF_2$ |

| Het | $R^1$ | $R^2$ | $R^3$ |
|---|---|---|---|
| 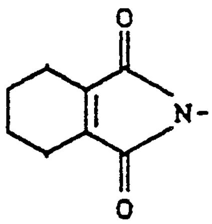 | F | $-(CH_2)_2-CH_3$ | $-CH_2-COOCH_2-CH_2-OCH_3$ |
| 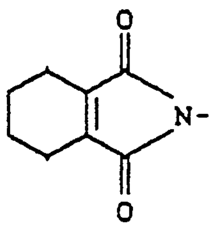 | F | $-(CH_2)_2-CH_3$ | $-\overset{\overset{CH_3}{\vert}}{C}H-COOC_2H_5$ |
| 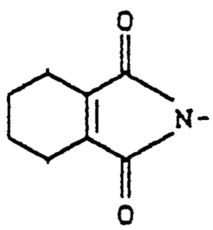 | F | $-(CH_2)_2-CH_3$ | $-CF_2-CHF-CHClF$ |
| 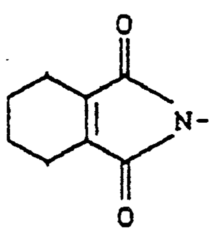 | F | $-(CH_2)_2-CH_3$ | $-CH(CH_3)_2$ |
| 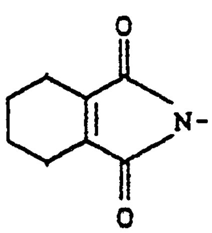 | F | $-(CH_2)_2-CH_3$ | $-CH_2-C\equiv C-CH_3$ |

Verwendet man beispielsweise 3,4,5,6-Tetrahydrophthalsäureanhydrid und 2-Fluor-4-methyl-5-difluormethoxyanilin als Ausgangsstoffe, so läßt sich der Reaktionsablauf des erfindungsgemäßen Verfahrens (a) durch das folgende Formelschema darstellen:

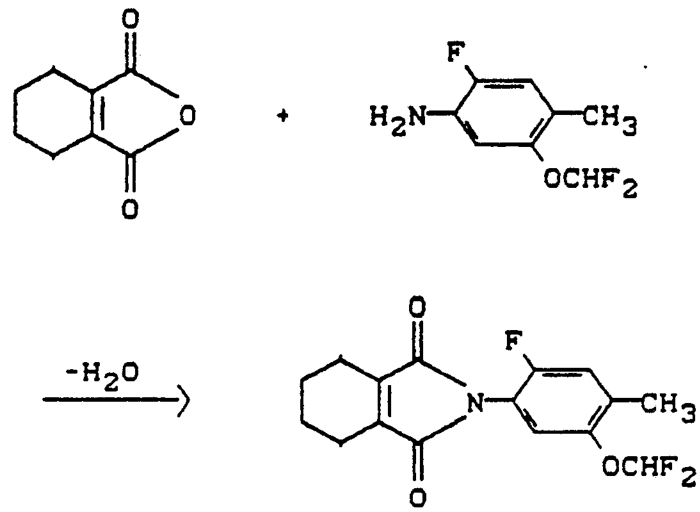


Verwendet man beispielsweise N-(2-Fluor-4-methyl-5-isopropoxy-phenyl)-3,4,5,6-tetrahydrophthalimid als Ausgangsverbindung und Natriumborhydrid als Reduktionsmittel, so läßt sich der Reaktionsablauf des erfindungsgemäßen Verfahrens (b) durch das folgende Formelschema darstellen:

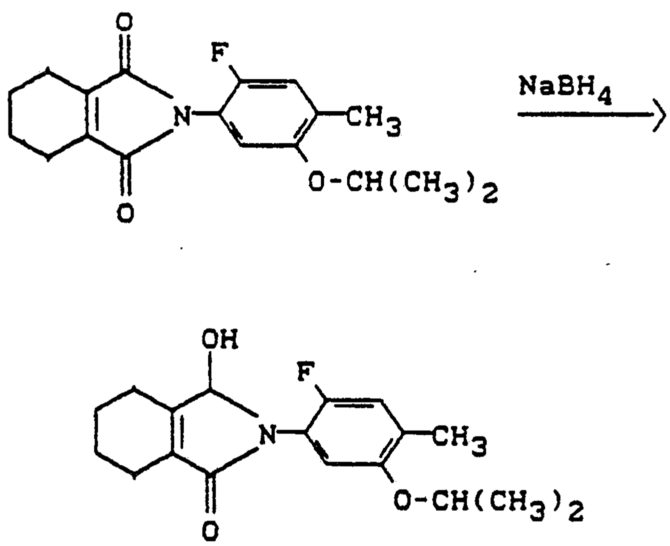


Verwendet man beispielsweise N-(2-Fluor-4-methyl-5-allyloxy-phenyl)-3,4,5,6-tetrahydrophthalimid und "Lawessons Reagenz" als Ausgangsstoffe, so läßt sich der Reationsablauf des erfindungsgemäßen Verfahrens (c) durch das folgende Formelschema darstellen:

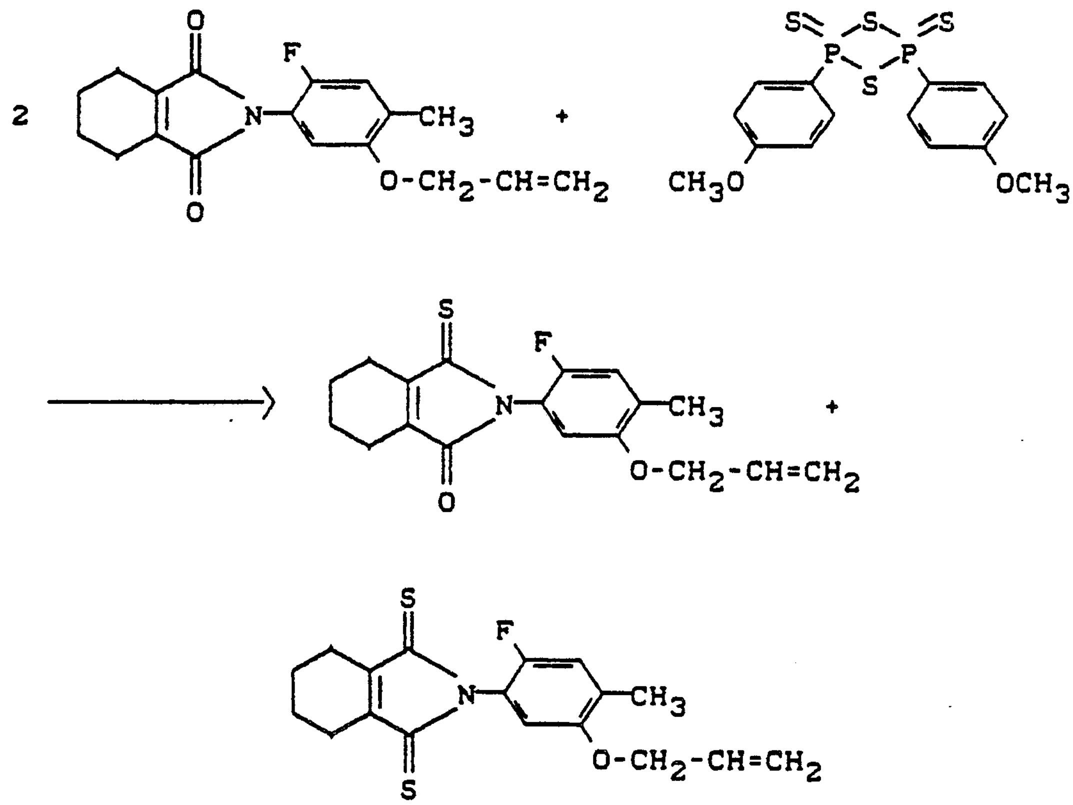

Verwendet man beispielsweise N-(2-Fluor-4-methyl-5-methansulfonyloxy-phenyl)-3,4-dimethyl-$\Delta^3$-pyrrolin-5-ol-2-on und Thionylchlorid als Ausgangsstoffe, so läßt sich der Reaktionsablauf des erfindungsgemäßen Verfahrens (d) durch das folgende Formelschema darstellen:

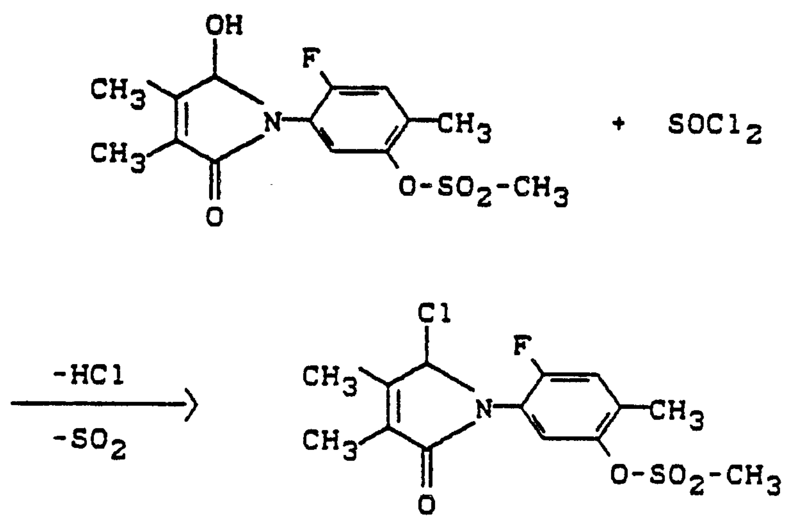


Verwendet man beispielsweise N-(2-Fluor-4-methyl-5-isopropyloxy-phenyl)-3,4-dimethyl-5-chlor-$\Delta^3$-pyrrolin-2-on als Ausgangsverbindung, so läßt sich der Reaktionsablauf des erfindungsgemäßen Verfahrens (e) durch das folgende Formelschema darstellen:

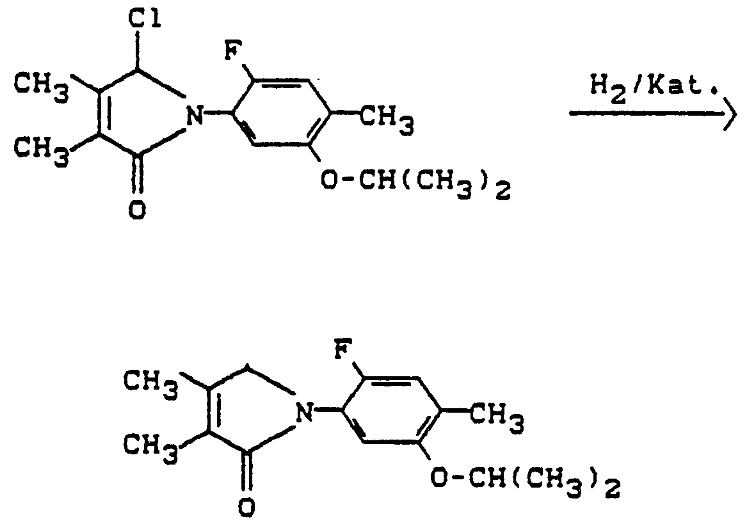


Verwendet man beispielsweise N-(2-Fluor-4-methyl-5-hydroxyphenyl)-3,4,5,6-tetrahydrophthalimid und Bromacetonitril als Ausgangsstoffe, so läßt sich der Reaktionsablauf des erfindungsgemäßen Verfahrens (f-α) durch das folgende Formelschema darstellen:

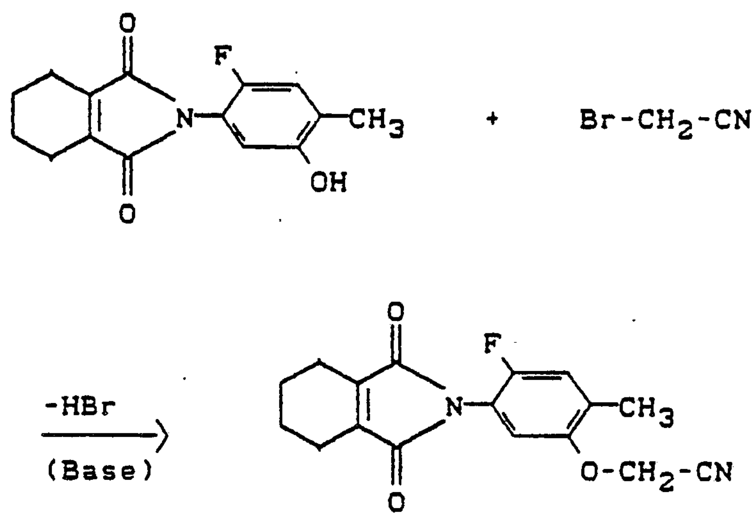


Verwendet man beispielsweise N-(2-Chlor-4-methyl-5-hydroxy-phenyl)-3,4-dimethyl-$\Delta^3$-pyrrolin-2,5-dion und Acetanhydrid als Ausgangsstoffe, so läßt sich der Reaktionsablauf des erfindungsgemäßen Verfahrens (f-β) durch das folgende Formelschema darstellen:

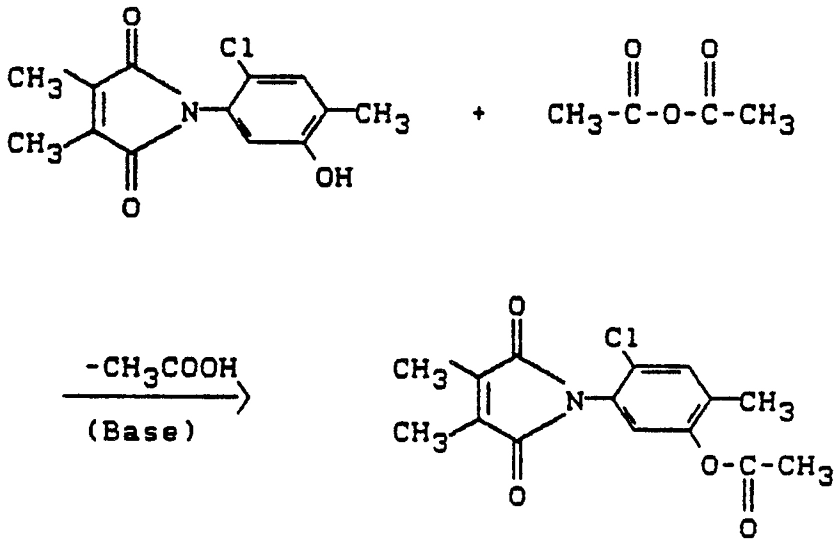


Verwendet man beispielsweise N-(2-Fluor-4-methyl-5-hydroxy-phenyl)-$\Delta^3$-pyrrolin-2,5-dithion und Methansulfonsäurechlorid als Ausgangsstoffe, so läßt sich der Reaktionsablauf des erfindungsgemäßen Verfahrens (f-γ) durch das folgende Formelschema darstellen:

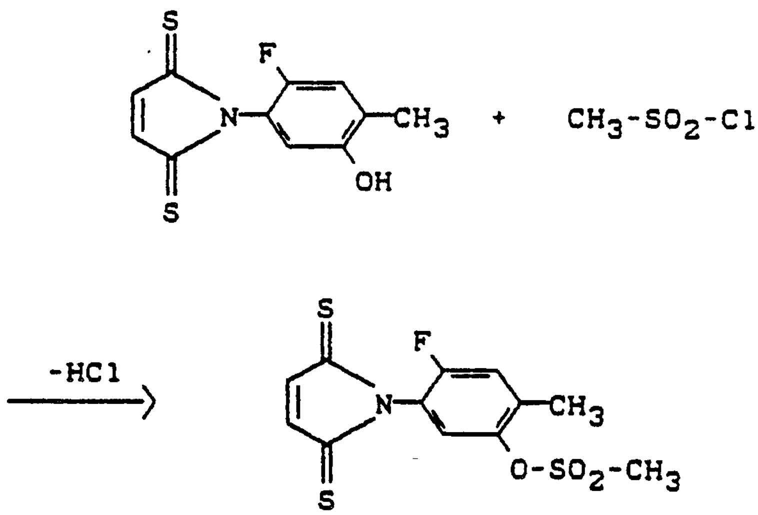


Die zur Durchführung des erfindungsgemäßen Verfahrens (a) als Ausgangsstoffe benötigten Anhydride sind durch die Formel (II) allgemein definiert. In dieser Formel (II) steht A vorzugsweise für diejenigen Reste, die bereits im Zusammenhang mit der Beschreibung der erfindungsgemäßen Stoffe der Formel (I) als bevorzugt für diesen Substituenten genannt wurden.

Die Anhydride der Formel (II) sind allgemeine bekannte Verbindungen der organischen Chemie oder erhältlich in Analogie zu allgemein bekannten Verfahren (vgl. z.B. Gazz. chim. ital. 57, 300-311 [1927]).

Die zur Durchführung des erfindungsgemäßen Verfahrens (a) weiterhin als Ausgangsstoffe benötigten Amine sind durch die Formel (III) allgemein definiert. In dieser Formel (III) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind teilweise bekannt (vgl. z.B. Chem. Ber. 117, 2275 - 2286, [1984]; Bull. chem. Soc. Japan 49, 2294 - 2297, [1976]; Belg. Pat. BE 11 705 vom 06.09.1968 oder J. chem. Soc. 1965, 1187 -1191) oder können in Analogie zu bekannten Verfahren erhalten werden (vgl. z.B. EP 165 895 oder Roczniki Chem. 38, 51 - 59 [1964] bzw. CA 60 : 14 434 f).

Noch nicht bekannt sind Amine der Formel (IIIa),

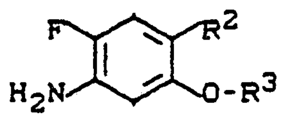

(IIIa)

in welcher
$R^2$ und $R^3$ die oben angegebene Bedeutung haben.

In der Formel (IIIa) haben $R^2$ und $R^3$ vorzugsweise bzw. besonders bevorzugt diejenigen Bedeutungen, die bereits bei der Beschreibung der erfindungsgemäßen Stoffe der Formel (I) vorzugsweise bzw. besonders bevorzugt für diese Reste genannt wurden.

Man erhält sie beispielsweise, wenn man fluorierte Phenolether der Formel (IX),

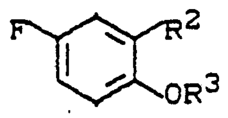

(IX)

in welcher
$R^2$ und $R^3$ die oben angegebene Bedeutung haben,
zunächst in einer 1. Stufe mit einem üblichen Nitrierungsmittel, wie beispielsweise Salpetersäure gegebenenfalls in Gegenwart eines Katalysators wie beispielsweise Schwefelsäure bei Temperaturen zwischen - 20 °C und + 30 °C nitriert, und die so erhältlichen Nitroverbindungen der Formel (X),

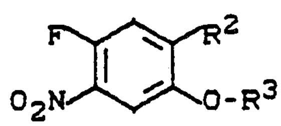

(X)

in welcher
$R^2$ und $R^3$ die oben angegebene Bedeutung haben,
mit üblichen Reduktionsmitteln wie beispielsweise molekularem Wasserstoff in Gegenwart eines Katalysators wie beispielsweise Raney-Nickel gegebenenfalls in Gegenwart eines Verdünnungsmittels wie beispielsweise Tetrahydrofuran bei Temperaturen zwischen 0 °C und 80 °C und einem Druck zwischen 1 und 60 bar in allgemein üblicher Art und Weise reduziert.

Fluorierte Phenolether der Formel (IX) sind teilweise bekannt (vgl. z.B. Liebigs Ann. Chem. 595, 131 - 159 [1955]; J. Amer. chem. Soc. 61, 161 - 165 [1939] oder Bull. acad. Polon. Sci.; Sci. Chim. 11, 117 - 120 [1963] bzw. CA 59; 7408a) oder erhältlich in Analogie zu allgemein bekannten Verfahren, beispielsweise, wenn man fluorierte Phenole der Formel (XI),

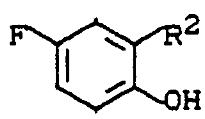

(XI)

in welcher
$R^2$ die oben angegebene Bedeutung hat,
in allgemein üblicher Art und Weise alkyliert, acyliert oder sulfonyliert (vgl. hierzu die Angaben zu den erfindungsgemäßen Verfahren (f-α), (f-β) und (f-γ) sowie die Herstellungsbeispiele).

Fluorierte Phenole der Formel (XI) sind bekannt (vgl. z.B. EP 18 606; Chem. Ber. 86, 501 - 507 [1953]; J. Amer. chem. Soc. 61, 161 - 165 [1939]).

Nitroverbindungen der Formel (X) sind neu. In der Formel (X) haben $R^2$ und $R^3$ vorzugsweise bzw. besonders bevorzugt diejenigen Bedeutungen, die bereits bei der Beschreibung der erfindungsgemäßen Stoffe der Formel (I) vorzugsweise bzw. besonders bevorzugt für diese Reste genannt wurden.

Nitroverbindungen der Formel (X) erhält man alternativ auch, wenn man fluorierte Nitrophenole der Formel (XII),

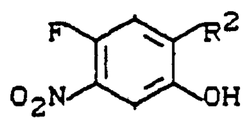

(XII)

in welcher
$R^2$ die oben angegebene Bedeutung hat,
in allgemein üblicher Art und Weise alkyliert, acyliert oder sulfonyliert (vgl. hierzu die Angaben zu den erfindungsgemäßen Verfahren (f-α), (f-β) und (f-γ) sowie die Herstellungsbeispiele).

Fluorierte Nitrophenole der Formel (XII) sind noch nicht bekannt. Man erhält sie, wenn man fluorierte Phenole der Formel (XI),

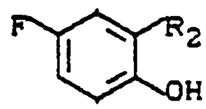

(XI)

in welcher
$R^2$ die oben angegebene Bedeutung hat,
zunächst in allgemein bekannter Weise mit Chlorameisensäuremethylester gegebenenfalls in Gegenwart einer Base wie beispielsweise Natriumhydroxid an der phenolischen OH-Gruppe acyliert, dann in einer 2. Stufe die so erhältlichen Phenylcarbonate der Formel (IXa),

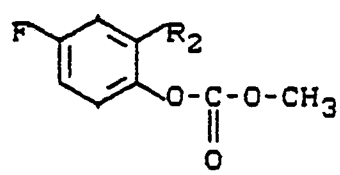

(IXa)

in welcher
$R^2$ die oben angegebene Bedeutung hat,
mit Salpetersäure gegebenenfalls in Gegenwart eines Katalysators wie beispielsweise Schwefelsäure bei Temperaturen zwischen 0 °C und 50 °C nitriert und schließlich in einer 3. Stufe die Nitrophenylcarbonate der Formel (Xa),

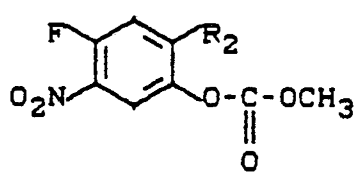

(Xa)

in welcher
$R^2$ die oben angegebene Bedeutung hat,
mit Salzsäure gegebenenfalls in Gegenwart eines Verdünnungsmittels wie beispielsweise Dioxan bei Temperaturen zwischen 50 °C und 120 °C deacyliert.

Die zur Durchführung der erfindungsgemäßen Verfahren (b) und (c) als Ausgangsstoffe benötigten N-Arylstickstoffheterocyclen sind durch die Formel (Ia) allgemein definiert. In dieser Formel (Ia) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Het[1] steht vorzugsweise für einen Rest

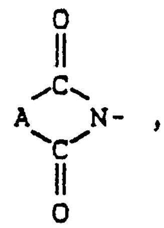

wobei A vorzugsweise für diejenigen Reste steht, die bereits im Zusammenhang mit der Beschreibung der erfindungsgemäßen Stoffe der Formel (I) als bevorzugt für diesen Substituenten genannt wurden.

Die N-Arylstickstoffheterocyclen sind erfindungsgemäße Verbindungen und erhältlich mit Hilfe des erfindungsgemäßen Verfahrens (a).

Das zur Durchführung des erfindungsgemäßen Verfahrens (c) weiterhin als Ausgangsstoff benötigte "Lawesson Reagenz" ist durch die Formel (IV) definiert.

Das "Lawesson Reagenz" der Formel (IV) ist bekannt (vgl. z.B. DE-OS 2 606 083; Bull. Soc. Chim. Belg. 87, 223-228 [1978]).

Die zur Durchführung des erfindungsgemäßen Verfahrens (d) als Ausgangsstoffe benötigten N-Aryl-Stickstoffheterocyclen sind durch die Formel (Ib) allgemein definiert. In dieser Formel (Ib) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

$Het^2$ steht vorzugsweise für einen Rest

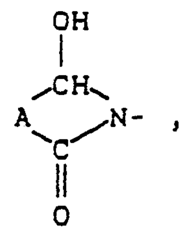

wobei A vorzugsweise für diejenigen Reste steht, die bereits im Zusammenhang mit der Beschreibung der erfindungsgemäßen Stoffe der Formel (I) als bevorzugt für diesen Substituenten genannt wurden.

Die N-Aryl-Stickstoffheterocyclen der Formel (Ib) sind erfindungsgemäße Verbindungen und erhältlich mit Hilfe des erfindungsgemäßen Verfahrens (b).

Die zur Durchführung des erfindungsgemäßen Verfahrens (e) als Ausgangsstoffe benötigten N-Aryl-Stickstoffheterocyclen sind durch die Formel (Id) allgemein definiert. In dieser Formel (Id) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

$Het^4$ steht vorzugsweise für einen Rest

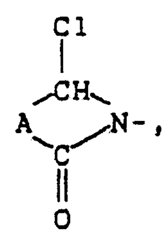

wobei A vorzugsweise für diejenigen Reste steht, die bereits im Zusammenhang mit der Beschreibung der erfindungsgemäßen Stoffe der Formel (I) als bevorzugt für diesen Substituenten genannt wurden.

Die N-Aryl-Stickstoffheterocyclen der Formel (Id) sind erfindungsgemäße Verbindungen und erhältlich mit Hilfe des erfindungsgemäßen Verfahrens (d).

Die zur Durchführung der erfindungsgemäßen Verfahren (f-$\alpha$), (f-$\beta$) und (f-$\gamma$) als Ausgangsstoffe benötigten Phenole sind durch die Formel (V) allgemein definiert. In dieser Formel (V) stehen $R^1$ und $R^2$

vorzugsweise für diejenigen Reste, die bereits im Zusammenhang mit der Beschreibung der erfindungsgemäßen Stoffe der Formel (I) als bevorzugt für diese Substituenten genannt wurden.

Het[1] steht vorzugsweise für einen Rest

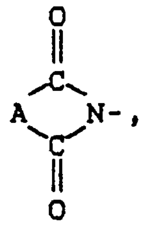

wobei

A vorzugsweise für diejenigen Reste stehen, die bereits im Zusammenhang mit der Beschreibung der erfindungsgemäßen Stoffe der Formel (I) als bevorzugt für diesen Substituenten genannt wurden.

Die Phenole der Formel (V) sind teilweise bekannt (vgl. z.B. Monatshefte für Chemie, 95, 392-397 [1964] bzw. CA 61 : 5546c).

Noch nicht bekannt sind Phenole der Formel (Va),

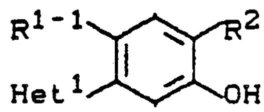

(Va)

wobei

$R^{1-1}$ für Halogen, insbesondere für Fluor oder Chlor steht und

$R^2$ und $Het^1$ die oben angegebene Bedeutung haben.

In der Formel (Va) haben $R^2$ und $Het^1$ vorzugsweise bzw. besonders bevorzugt diejenigen Bedeutungen, die bereits bei der Beschreibung der erfindungsgemäßen Stoffe der Formel (I) bzw. (Ia) bevorzugt bzw. besonders bevorzugt für diese Reste genannt wurden, $R^{1-1}$ steht insbesondere für Fluor oder Chlor.

Man erhält sie in Analogie zu bekannten Verfahren, wenn man Nitrophenole der Formel (XIIa),

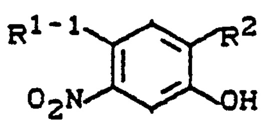

(XIIa)

in welcher

$R^{1-1}$ und $R^2$ die oben angegebene Bedeutung haben,

mit üblichen Reduktionsmitteln, wie beispielsweise molekularem Wasserstoff in Gegenwart eines geeigneten Katalysators wie beispielsweise Raney-Nickel oder Platinoxid gegebenenfalls in Gegenwart eines Verdünnungsmittels wie beispielsweise Tetrahydrofuran oder Ethanol bei Temperaturen zwischen 0 °C und 80 °C und einem Druck zwischen 1 und 50 bar in allgemein üblicher Art und Weise reduziert und die so erhältlichen Aminophenole der Formel (XIII),

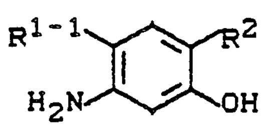

(XIII)

in welcher

$R^{1-1}$ und $R^2$ die oben angegebene Bedeutung haben,

mit Anhydriden der Formel (II),

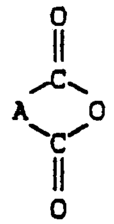

(II)

in welcher
A die oben angegebene Bedeutung hat,
in Analogie zur Durchführung des erfindungsgemäßen Verfahrens (a) umsetzt.

Nitrophenole der Formel (XIIa), bei welchen $R^{1-1}$ für Chlor steht sind bekannt (vgl. z.B. GB 1 100 219).

Die zur Durchführung des erfindungsgemäßen Verfahrens (f-α) weiterhin als Ausgangsstoffe benötigten Alkylierungsmittel sind durch die Formel (VI) allgemein definiert. In dieser Formel (VI) steht $R^{3-1}$ vorzugsweise für diejenigen Reste, die bereits im Zusammenhang mit der Beschreibung der erfindungsgemäßen Stoffe der Formel (I) als bevorzugt für den Substituenten $R^3$ genannt wurden, mit Ausnahme der Reste - $\underset{O}{\underset{\|}{C}}$ - $R^4$ sowie $-SO_2-R^5$.

$R^{3-1}$ steht besonders bevorzugt für Methyl, Ethyl, n-oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor, Chlor oder Brom, für jeweils geradkettiges oder verzweigtes Cyanalkyl, Alkoxyalkyl, Alkoxyalkoxyalkyl oder Alkoxycarbonylalkyl mit jeweils 1 bis 3 Kohlenstoffatomen in den einzelnen Alkylteilen, außerdem für jeweils gegebenenfalls ein- bis sechsfach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclobutenyl, Cyclopenthyl, Cyclohexenyl, Cyclobutyl, Cyclopropyl, Cyclopentyl oder Cyclohexyl, außerdem für gegebenenfalls einfach durch Methyl oder Ethyl substituiertes Oxetanylmethyl, Tetrahydrofuranylmethyl oder Tetrahydropyranylmethyl oder für jeweils gegebenenfalls ein- bis dreifach gleich oder verschieden substituiertes Benzyl oder Phenylethyl, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio.

$E^1$ steht vorzugsweise für Halogen insbesondere für Brom oder Iod, für Alkoxysulfonyloxy oder für gegebenenfalls substituiertes Arylsulfonyloxy wie beispielsweise Methoxysulfonyloxy, Ethoxysulfonyloxy oder p-Toluolsulfonyloxy.

Die Alkylierungsmittel der Formel (VI) sind allgemein bekannte Verbindungen der organischen Chemie.

Die zur Durchführung des erfindungsgemäßen Verfahrens (f-β) weiterhin als Ausgangsstoffe benötigten Acylierungsmittel sind durch die Formel (VII) allgemein definiert. In dieser Formel (VII) steht $R^4$ vorzugsweise für diejenigen Reste, die bereits im Zusammenhang mit der Beschreibung der erfindungsgemäßen Stoffe der Formel (I) als bevorzugt für diesen Substituenten genannt wurden.

$E^2$ steht vorzugsweise für Halogen, insbesondere für Chlor oder Brom oder für einen Rest -O- $\underset{O}{\underset{\|}{C}}$ -$R^4$,
wobei $R^4$ die oben angegebene Bedeutung hat.

Die Acylierungsmittel der Formel (VII) sind allgemein bekannte Verbindungen der organischen Chemie.

Die zur Durchführung des erfindungsgemäßen Verfahrens (f-γ) weiterhin als Ausgangsstoffe benötigten Sulfonylierungsmittel sind durch die Formel (VIII) allgemein definiert. In dieser Formel (VIII) steht $R^5$ vorzugsweise für diejenigen Reste, die bereits im Zusammenhang mit der Beschreibung der erfindungsgemäßen Stoffe der Formel (I) als bevorzugt für diesen Substituenten genannt wurden.

$E^2$ steht vorzugsweise für Halogen, insbesondere für Fluor, Chlor oder Brom.

Die Sulfonylierungsmittel der Formel (VIII) sind ebenfalls allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische,

gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Carbonsäuren, wie Essigsäure oder Propionsäure.

Das erfindungsgemäße Verfahren (a) kann gegebenenfalls in Gegenwart eines geeigneten Reaktionshilfsmittels durchgeführt werden. Vorzugsweise verwendet man anorganische oder organische Säuren wie beispielsweise Essigsäure oder p-Toluolsulfonsäure, Anhydride wie beispielsweise Acetanhydrid oder Säurechloride wie Acetylchlorid als Reaktionshilfsmittel. Es ist auch möglich andere übliche wasserabspaltende Mittel wie beispielsweise N,N′-Dicyclohexylcarbodiimid oder übliche Acylierungskatalysatoren, wie beispielsweise 4-(N,N-Dimethylamino)-pyridin als Reaktionshilfsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 180 °C, vorzugsweise bei Temperaturen zwischen 50 °C und 150 °C.

Zur Durchführung des erfindungsgemäßen Verfahrens (a) setzt man pro Mol an Anhydrid der Formel (II) im allgemeinen 1.0 bis 1.5 Mol, vorzugsweise 1.0 bis 1.2 Mol an Amin der Formel (III) und gegebenenfalls 0.01 bis 1.2 Mol, vorzugsweise 0.1 bis 1.0 Mol an Reaktionshilfsmittel ein. Die Reaktionsdurchführung, Aufarbeitung und Isolierung der Reaktionsprodukte erfolgt nach allgemein üblichen Methoden.

Als Reduktionsmittel zur Durchführung des erfindungsgemäßen Verfahrens (b) kommen alle üblichen für derartige Reduktionsreaktionen verwendbaren Reduktionsmittel infrage. Vorzugsweise verwendet man komplexe Hydride, wie beispielsweise Natriumborhydrid, Natriumcyanoborhydrid, Lithiumborhydrid oder Lithiumaluminiumhydrid.

Als Verdünnungsmittel zur Durchführung des erfindungsgemäßen Verfahrens (b) kommen in Abhängigkeit vom verwendeten Reduktionsmittel alle üblichen organischen oder anorganischen Lösungsmittel infrage. Vorzugsweise verwendet man Ether, wie Diethylether, Dioxan oder Tetrahydrofuran oder Alkohole, wie Methanol, Ethanol oder Propanol.

Die Reaktionstemperaturen können bei der Durchführung des erfindungsgemäßen Verfahrens (b) in Abhängigkeit vom verwendeten Reduktionsmittel in einem größeren Bereich variiert werden. Im allgemeinen arbeitet man bei Temperaturen zwischen - 20 °C und. + 150 °C, vorzugsweise bei Temperaturen zwischen 0 °C und 120 °C.

Zur Durchführung des erfindungsgemäßen Verfahrens (b) setzt man pro Mol an N-Aryl-Stickstoffheterocyclus der Formel (Ia) im allgemeinen 0.1 bis 2.0 Mol, vorzugsweise 0.25 bis 1.5 Mol an Reduktionsmittel ein.

Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether.

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 180 °C, vorzugsweise bei Temperaturen zwischen 20 °C und 150 °C.

Zur Durchführung des erfindungsgemäßen Verfahrens (c) setzt man pro Mol an N-Aryl-Stickstoffheterocyclus der Formel (Ia) im allgemeinen 0,2 bis 2.0 Mol, vorzugsweise 0,5 bis 1.5 Mol an "Lawesson Reagenz" der Formel (IV) ein.

Die Reaktionsdurchführung, Aufarbeitung und Isolierung der Reaktionsprodukte erfolgt in Analogie zu bekannten Verfahren (vgl. z.B. Bull. Soc. Chim. Belg. 87, 223-228 [1978]).

In der Regel erhält man bei der Durchführung des erfindungsgemäßen Verfahrens (c) als Endprodukt ein Gemisch aus Verbindungen der Formel (Ic-1),

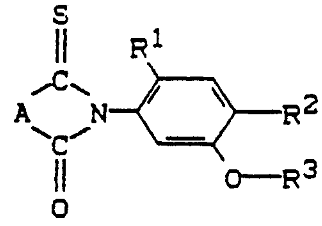

(Ic-1)

und der Formel (Ic-2),

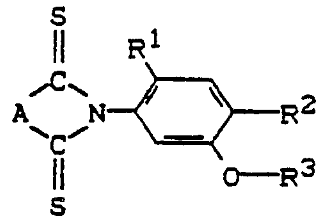

(Ic-2)

wobei
$R^1$, $R^2$, $R^3$ und A in beiden Formeln die oben angegebenen Bedeutungen haben.

Diese Gemische lassen sich mit üblichen Trennmethoden (Chromatographie, Kristallisation) auftrennen.

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Nitrile wie Acetonitril oder Propionitril.

Das erfindungsgemäße Verfahren (d) kann gegebenenfalls in Gegenwart eines geeigneten Reaktionshilfsmittels durchgeführt werden. Als solche kommen insbesondere organische Amine oder Amide infrage. Vorzugsweise verwendet man Pyridin, Dimethylanilin oder Dimethylformamid.

Die Reaktionstemperaturen können bei der Durchführung des erfindungsgemäßen Verfahrens (d) in einem größeren Bereich variiert werden. Im allgemeinen arbeitet man bei Temperaturen zwischen 0 °C und 120 °C, vorzugsweise bei Temperaturen zwischen 20 °C und 80 °C.

Zur Durchführung des erfindungsgemäßen Verfahrens (d) setzt man pro Mol an N-Aryl-Stickstoffheterocyclus der Formel (Ib) im allgemeinen 1.0 bis 5.0 Mol, vorzugsweise 1.0 bis 2.0 Mol an Thionylchlorid und gegebenenfalls 0.1 bis 2.0 Mol, vorzugsweise 0.5 bis 1.5 Mol an Reaktionshilfsmittel ein.
Die Reaktionsdurchführung, Aufarbeitung und Isolierung der Reaktionsprodukte erfolgt nach allgemein üblichen Methoden.

Als Katalysatoren zur Durchführung des erfindungsgemäßen Verfahrens (e) kommen alle üblichen Hydrierkatalysatoren infrage. Vorzugsweise verwendet man Edelmetallkatalysatoren, wie beispielsweise Platin, Platinoxid, Palladium oder Ruthenium gegebenenfalls auf einem geeignetem Träger wie beispielsweise Kohlenstoff oder Siliciumdioxid.

Als Verdünnungsmittel zur Durchführung des erfindungsgemäßen Verfahrens (e) kommen inerte organische Lösungsmittel infrage. Hierzu gehören insbesondere aliphatische oder alicyclische gegebenenfalls halogenierte Kohlenwasserstoffe, wie beispielsweise Benzin, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Alkohole wie Methanol, Ethanol oder Propanol.

Das erfindungsgemäße Verfahren (e) kann gegebenenfalls in Gegenwart eines geeigneten Säurebindemittels durchge führt werden. Vorzugsweise verwendet man Alkalimetallcarbonate wie Natriumcarbonat oder Kaliumcarbonat oder organische Basen wie Pyridin oder Lutidin.

Die Reaktionstemperaturen können bei der Durchführung des erfindungsgemäßen Verfahrens (e) in einem größeren Bereich variiert werden. Im allgemeinen arbeitet man bei Temperaturen zwischen - 20 °C und 100 °C, vorzugsweise bei Temperaturen zwischen 0 °C und 50 °C.

Das erfindungsgemäße Verfahren kann bei Normaldruck oder unter erhöhtem Druck durchgeführt werden. Vorzugsweise arbeitet man unter Normaldruck.

Zur Durchführung des erfindungsgemäßen Verfahrens (e) setzt man pro Mol an N-Aryl-Stickstoffheterocyclus der Formel (Id) im allgemeinen 1.0 bis 3.0 Mol, vorzugsweise 1.0 bis 1.5 Mol an Wasserstoff und gegebenenfalls 1.0 bis 3.0 Mol, vorzugsweise 1.0 bis 1.5 Mol an Säurebindemittel ein.
Die Reaktionsdurchführung, Aufarbeitung und Isolierung der Reaktionsprodukte erfolgt nach allgemein üblichen Methoden.

Zur Durchführung des erfindungsgemäßen Verfahrens (f) kommen als Verdünnungsmittel inerte organische Lösungsmittel infrage. Vorzugsweise verwendet man aliphatische, alicyclische oder aromatische, gegebenenfalls halogenierte Kohlenwasserstoffe, wie beispielsweise Benzin, Benzol, Toluol, Xylol, Pentan, Hexan, Heptan, Cyclo hexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Säuren, wie Essigsäure, Nitrile, wie Acetonitril oder Propionitril, Amide, wie Dimethylformamid, Dimethylacetamid, N-Methylpyrrolidon oder Hexamethylphosphorsäuretriamid. Verwendet man als Reaktionspartner in den Varianten (f-$\alpha$), (f-$\beta$) oder (f-$\gamma$) Verbindungen der Formeln (VI), (VII) oder (VIII) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (f) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (f) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (f) setzt man pro Mol an Phenol der Formel (V) in den Varianten (f-$\alpha$), (f-$\beta$) oder (f-$\gamma$) im allgemeinen jeweils 1,0 bis 20,0 Mol, vorzugsweise jeweils 1,0 bis 15,0 Mol, an Alkylierungsmittel der Formel (VI) oder Acylierungsmittel der Formel (VII) oder Sulfonylierungsmittel der Formel (VIII) und gegebenenfalls 1,0 bis 3,0 Mol, vorzugsweise 1,0 bis 2,0 Mol an Reaktionshilfsmittel, ein. Die Reaktionsdurchführung, Aufarbeitung und Isolierung der Reaktionsprodukte der Formel (If) erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Cheno podium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur Bekämpfung von mono- und dikotylen Unkräutern in mono- und dikotylen Kulturen, wie beispielsweise Soja, Weizen oder Sonnenblumen einsetzen.

Auch die Zwischenprodukte der Formel (V) besitzen gute herbizide Wirksamkeit.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen in wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten in allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (Amethydione) oder N-(2-Benzthiazolyl)-N,N′-dimethyl-harnstoff (Metabenzthiazuron) zur Unkrautbekämpfung in Getreide; 4-Amino-3-methyl-6-phenyl-1,2,4-triazin-5(4H)-on zur (Metamitron) Unkrautbekämpfung in Zuckerrüben und 4-Amino-6-(1,1-dimethylethyl)-3-methylthio-1,2,4-triazin-5(4H)-on (Metribuzin) zur Unkrautbe kämpfung in Sojabohnen, infrage. Auch Mischungen mit
2,4-Dichlorphenoxyessigsäure (2,4-D);
2,4-Dichlorphenoxypropionsäure (2,4-DP);
4-(2,4-Dichlorphenoxy)-buttersäure (2,4-DB);
(2-Methyl-4-chlorphenoxy)-essigsäure (MCPA);
(4-Chlor-2-methylphenoxy)-propionsäure (MCPP);
3,5,6-Trichlor-2-pyridyloxyessigsäure (TRICLOPYR);
2-[4-(2,4-Dichlorphenoxy)-phenoxy]-propionsäure, deren Methyl- oder deren Ethylester (DICLOFOP-[METHYL]);
2-[4-(3,5-Dichlorpyrid-2yloxy)-phenoxy]-propionsäure-(trimethylsilylmethylester);
3,6-Dichlor-2-pyridincarbonsäure (CLOPYRALID);
Chloressigsäure-N-(methoxymethyl)-2,6-diethylanilid (ALACHLOR);
2-Ethyl-6-methyl-N-(1-methyl-2-methoxyethyl)-chloracetanilid (METOLACHLOR);
N-(1-Ethylpropyl)-3,4-dimethyl-2,6-dinitroanilin (PENDIMETHALIN);
2,6-Dinitro-4-trifluormethyl-N,N-dipropylanilin (TRIFLURALIN);
N,N-Dimethyl-N′-(3-chlor-4-methylphenyl)-harnstoff (CHLORTOLURON);
N,N-Dimethyl-N′-(4-isopropylphenyl)-harnstoff (ISOPROTURON);
2-[4,5-Dihydro-4-methyl-4-isopropyl-5-oxo-(1H)-imidazol-2-yl]-5-ethyl-pyridin-3-carbonsäure (IMAZETHAPYR);
Methyl-2-[4,5-dihydro-4-methyl-4-(1-methylethyl)-5-oxo-1H-imidazol-2-yl]-4,(5)-methylbenzoat (IMAZAMETHABENZ);
2-[5-Methyl-5-(1-methylethyl)-4-oxo-2-imidazolin-2-yl]-3-chinolincarbonsäure (IMAZAQUIN);
3,5-dibrom-4-hydroxy-benzonitril (BROMOXYNIL);
3,5-Diiod-4-hydroxybenzonitril (IOXYNIL);
N-Methyl-2-(1,3-benzthiazol-2-yloxy)-acetanilid (MEFENACET);
2-[[[[(4,6-Dimethoxypyrimidin-2-yl)-amino]-carbonyl]-amino]-sulfonyl]-methyl]-benzoesäuremethylester, (BENSULFURON);
Ethyl-2-{[(4-chlor-6-methoxy-2-pyrimidinyl)-aminocarbonyl]-aminosulfonyl}-benzoat (CHLORIMURON);
2-Chlor-N-{[(4-methoxy-6-methyl-1,3,5-triazin-2-yl)-amino]-carbonyl}-benzolsulfonamid (CHLORSULFURON);
2-{[[((4-Methoxy-6-methyl-1,3,5-triazin-2-yl)-amino)-carbonyl]-amino]-sulfonyl}-benzoesäure oder deren Methylester (METSULFURON);
3-[[[[(4-Methoxy-6-methyl-1,3,5-triazin-2-yl)-amino]-carbonyl]-amino]-sulfonyl]-thiophen-2-carbonsäuremethylester (THIAMETHURON);
N,S-Diethyl-N-cyclohexyl-thiolcarbamat (CYCLOATE);
4-Ethylamino-2-t-butylamino-6-methylthio-s-triazin (TERBUTRYNE);
3-Isopropyl-2,1,3-benzothiadiazin-4-on-2,2-dioxid (BENTAZON);
4-Amino-6-t-butyl-3-ethylthio-1,2,4-triazin-5(4H)-on (ETHIOZIN);
exo-1-Methyl-4-(1-methylethyl)-2-(2-methylphenyl-methoxy)-7-oxabicyclo-(2,2,1)-heptan (CINMETHYLIN) und
O-(6-Chlor-3-phenyl-pyridazin-4-yl)-S-octyl-thiocarbonat (PYRIDATE) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmittel ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B> durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann bei der Anwendung als Herbizide in einem größeren Bereich schwanken. Sie hängt im wesentlichen von der Art der gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die erfindungsgemäßen Wirkstoffe können bei der Anwendung als Wachstumregulatoren in den Formulierungen ebenfalls in Mischung mit anderen bekannten Wirkstoffen vorliegen, wie Fungizide, Insektizide, Akarizide und Herbizide sowie in Mischungen mit Düngemitteln und anderen Wachstumsregulatoren.

Die Wirkstoffe können dabei als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können bei der Anwendung als Wachstumsregulatoren ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

## Herstellungsbeispiele

### Beispiel 1

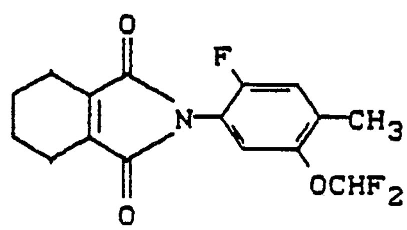

(Verfahren a)

11,5 g (0,073 Mol) 3,4,5,6-Tetrahydrophthalsäureanhydrid, 14,0 g (0,073 Mol) 5-Difluormethoxy-2-fluor-4-methylanilin und 0,9 g 4-(N,N-Dimethylamino)-pyridin in 50 ml Eisessig werden 3,5 Stunden auf 80 °C erwärmt. Das erkaltete Reaktionsgemisch wird in 400 ml Wasser gegeben, mit Dichlormethan extrahiert, die vereinigten organischen Phasen mit Wasser gewaschen, über Natriumsulfat getrocknet und im Vakuum eingeengt.

Man erhält 19,2 g (80 % der Theorie) an N-(5-Difluormethoxy-2-fluor-4-methyl-phenyl)-3,4,5,6-tetrahydrophthalimid als zähes Öl.

$^1$H-NMR ($CDCl_3$) : $\delta$ = 6,47 (t,1H,-OCHF$_2$) ppm.

### Beispiel 2

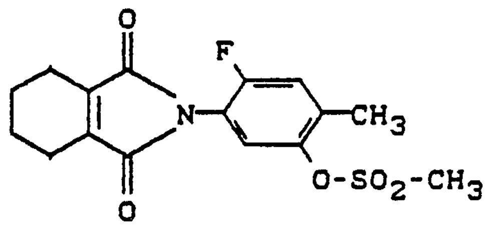

(Verfahren a)

3,8 g (0,024 Mol) 3,4,5,6-Tetrahydrophthalsäureanhydrid und 5,0 g (0,023 Mol) 5-Amino-4-fluor-2-methyl-phenyl-methansulfonsäureester in 30 ml Eisessig werden 2 Stunden auf 80 °C erwärmt, abgekühlt und in 300 ml Wasser gegeben; der kristalline Niederschlag wird abgesaugt und getrocknet.

Man erhält 5,9 g (73 % der Theorie) an N-(2-Fluor-5-methansulfonyloxy-4-methyl-phenyl)-3,4,5,6-tetrahydrophthalimid vom Schmelzpunkt 129 °C - 131 °C.

Beispiel 3

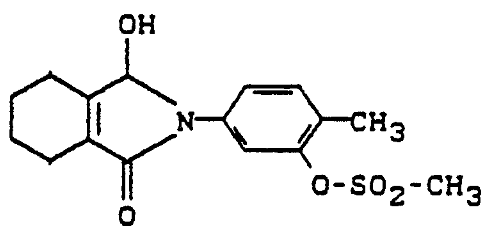

(Verfahren b)

7 g (0,021 Mol) NM-(5-Methansulfonyloxy-4-methyl-phenyl)-3,4,5,6-tetrahydrophthalimid in 100 ml Methanol werden bei 20 °C portionsweise mit 1,2 g (0,031 Mol) Natriumborhydrid versetzt, anschließend 2 Stunden bei Raumtemperatur gerührt und dann in 300 ml Wasser gegeben; der kristalline Niederschlag wird abgesaugt und im Vakuum getrocknet.

Man erhält 6,2 g (88% der Theorie) an N-(5-Methansulfonyloxy-4-methyl-phenyl)-3-hydroxy-2,3,4,5,6,7-hexahydroisoindol-1-on vom Schmelzpunkt 152 °C -154 °C.

Beispiel 4

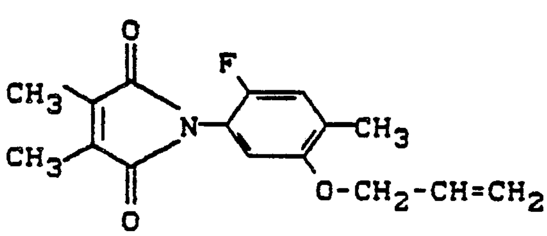

(Verfahren f-α)

2,72 g (0,012 Mol) N-(2-Fluor-5-hydroxy-4-methylphenyl)-3,4-dimethyl-$\Delta^3$-pyrrolin-2,5-dion, 2,07 g (0,015 Mol) Kaliumcarbonat und 1.35 ml (0,015 Mol) Allylbromid in 30 ml Acetonitril werden bei 60 °C so lange gerührt, bis im Dünnschichtchromatogramm kein Ausgangsprodukt mehr nachweisbar ist. Zur Aufarbeitung gibt man den Reaktionsansatz in 100 ml Wasser, extrahiert mit Dichlormethan, trocknet über Natriumsulfat, engt im Vakuum ein und kristallisert den Rückstand aus Essigester/n-Hexan um.

Man erhält 2,12 g (61 % der Theorie) an N-(5-Allyloxy-2-fluor-4-methyl-phenyl)-3,4-dimethyl-$\Delta^3$-pyrrolin-2,5-dion vom Schmelzpunkt 68 °C.

Beispiel 5

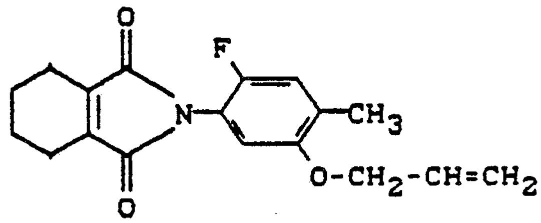

(Verfahren f-α)

2,37 g (0,009 Mol) N-(2-Fluor-5-hydroxy-4-methylphenyl)-3,4,5,6-tetrahydrophthalimid, 1,66 g (0,012 Mol) Kaliumcarbonat und 1,04 ml (0,012 Mol) Allylbromid in 30 ml Acetonitril werden bei 60 °C so lange gerührt, bis im Dünnschichtchromatogramm kein Ausgangsprodukt mehr nachweisbar ist. Zur Aufarbeitung wird filtriert, das Filtrat im Vakkum eingeengt, der Rückstand an Kieselgelchromatographiert (Laufmittel : Cyclohexan/Essigester 2:1) und aus Essigester/n-Hexan kristallisert.

Man erhält 1,79 g (63 % der Theorie) an N-(5-Allyloxy-2-fluor-4-methyl-phenyl)-3,4,5,6-tetrahydrophthalimid vom Schmelzpunkt 67 °C.

Beispiel 6

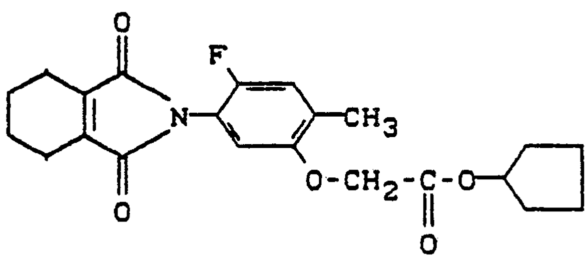

(Verfahren f-α)

2,37 g (0,009 Mol) N-(2-Fluor-5-hydroxy-4-methylphenyl)-3,4,5,6-tetrahydrophthalimid, 1,66 g (0,012 Mol) Kaliumcarbonat und 2,51 g (0,012 Mol) Bromessigsäurecyclopentylester in 30 ml absolutem Acetonitril werden bei 60 °C so lange gerührt bis im Dünnschichtchromatogramm kein Ausgangsprodukt mehr nachweisbar ist. Zur Aufarbeitung wird filtriert, das Filtrat im Vakuum eingeengt, der Rückstand an Kieselgel chromatographiert (Laufmittel: Cyclohexan/Essigester 2:1) und aus Essigester/n-Hexan kristallisiert.

Man erhält 1,46 g (43 % der Theorie) an N-(5-Cyclopentyloxycarbonylmethoxy-2-fluor-4-methyl-phenyl)-3,4,5,6-tetrahydrophthalimid vom Schmelzpunkt 94 °C.

In entsprechender Weise und gemäß den allgemeinen Angaben zur Herstellung erhält man die folgenden N-Aryl-Stickstoffheterocyclen der allgemeinen Formel (I):

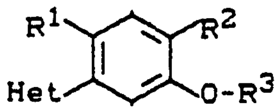

(I)

Tabelle 1

| Bsp. Nr. | Het | $R^1$ | $R^2$ | $R^3$ | physikalische Eigenschaften |
|---|---|---|---|---|---|
| 7 | 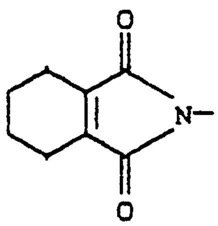 | F | $CH_3$ | $-CH_2C\equiv CH$ | Fp 134 °C |
| 8 | 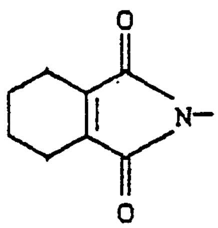 | F | $CH_3$ | $-CH_2-\overset{\overset{O}{\|}}{C}-O-(CH_2)_4-CH_3$ | Fp 46 °C |
| 9 | 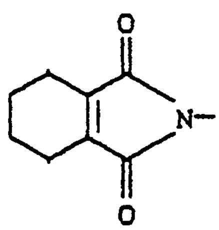 | F | $CH_3$ | $-\overset{\overset{CN}{\|}}{CH}-CH_3$ | Fp 96 °C |

Tabelle 1 (Fortsetzung)

| Bsp. Nr. | Het | $R^1$ | $R^2$ | $R^3$ | physikalische Eigenschaften |
|---|---|---|---|---|---|
| 10 | 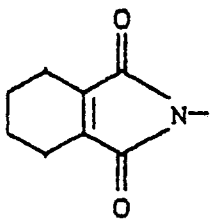 | F | CH | $-CH_2-CN$ | Fp 175 °C |
| 11 | 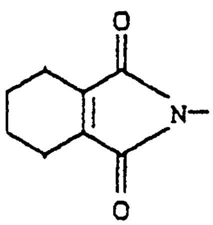 | F | $CH_3$ | $-CH(CH_3)_2$ | $^1$H-NMR*): 1,32; 2,2 |
| 12 | 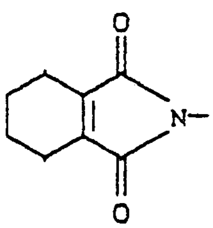 | F | $CH_3$ | 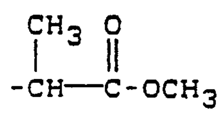 | Fp 70 °C |
| 13 | 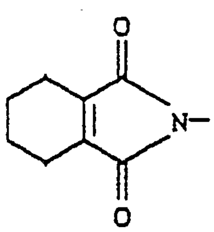 | F | $CH_3$ | 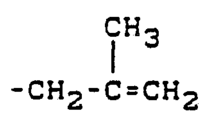 | Fp 85 °C |
| 14 | 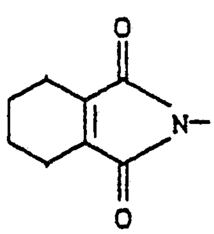 | F | $CH_3$ | 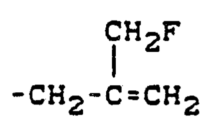 | Fp 70 °C |

Tabelle 1 (Fortsetzung)

| Bsp. Nr. | Het | $R^1$ | $R^2$ | $R^3$ | physikalische Eigenschaften |
|---|---|---|---|---|---|
| 15 | 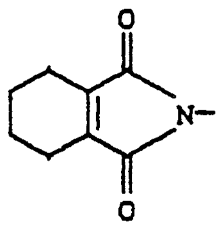 | F | $CH_3$ | 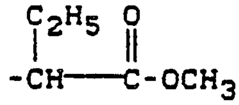 | $^1$H-NMR*): 2,3; 3,83 |
| 16 | 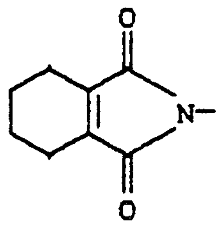 | F | $CH_3$ | 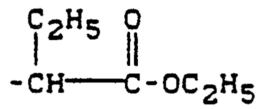 | $^1$H-NMR*): 1,22; 2,3; 4,19 |
| 17 | 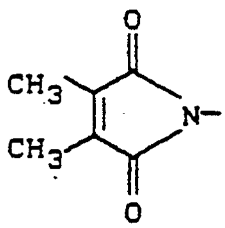 | F | $CH_3$ | 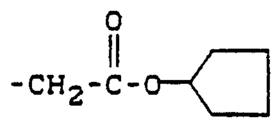 | Fp 99 °C |
| 18 | 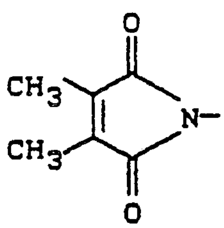 | F | $CH_3$ | 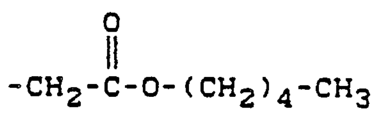 | Fp 58 °C |
| 19 | 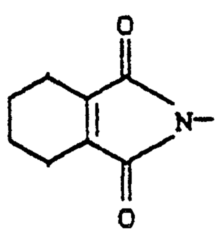 | H | $CH_3$ | $-SO_2-CH_3$ | Fp 99 °C - 103 °C |

Tabelle 1 (Fortsetzung)

| Bsp. Nr. | Het | $R^1$ | $R^2$ | $R^3$ | physikalische Eigenschaften |
|---|---|---|---|---|---|
| 20 | 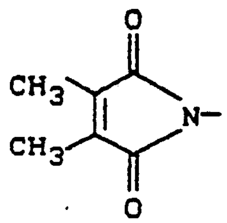 | F | $CH_3$ | $-CHF_2$ | Fp 107 °C-108 °C |
| 21 | 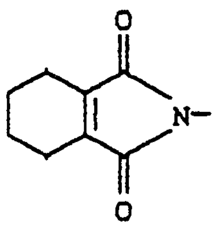 | F | $CH_3$ | $-SO_2-$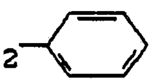 | Fp 128 °C-131 °C |
| 22 | 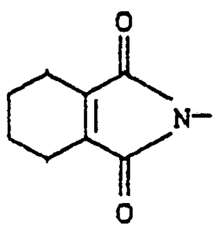 | F | $CH_3$ | $-CHF_2$ | Fp 85 °C-88 °C |
| 23 | 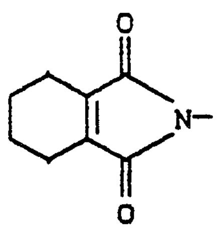 | F | $CH_3$ | $-SO_2-$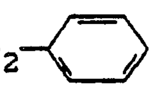 | Fp 128 °C-131 °C |
| 24 | 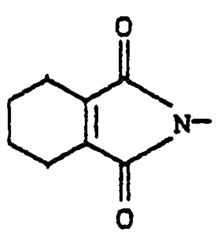 | F | $CH_3$ | $CF_3$<br>$-C=CH-CF_3$ | Fp 118 °C |

<u>Tabelle 1</u> (Fortsetzung)

| Bsp. Nr. | Het | $R^1$ | $R^2$ | $R^3$ | physikalische Eigenschaften |
|---|---|---|---|---|---|
| 25 | 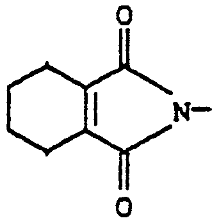 | F | $CH_3$ | $-CH(CH_2F)_2$ | Fp 100 °C |
| 26 | 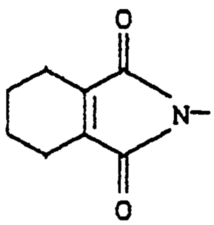 | H | $CH_3$ | $-CH_2-C \equiv CH$ | Fp 169 °C |
| 27 | 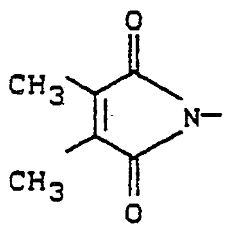 | F | $CH_3$ | $-CH_2-C \equiv CH$ | Fp 118 °C |
| 28 | 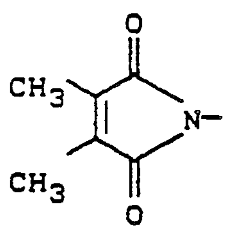 | F | $CH_3$ | $-CH-COOCH_3$<br>\|<br>$CH_3$ | Fp 95 °C |
| 29 | 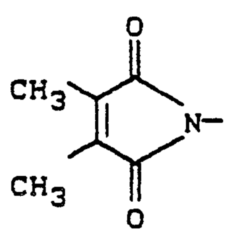 | F | $CH_3$ | $-CH-CH_3$<br>\|<br>$CN$ | Fp 100 °C |

Tabelle 1 (Fortsetzung)

| Bsp. Nr. | Het | $R^1$ | $R^2$ | $R^3$ | physikalische Eigenschaften |
|---|---|---|---|---|---|
| 30 | 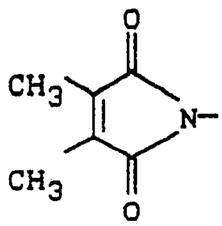 | F | $CH_3$ | $-CH_2CN$ | Fp 139 °C |
| 31 | 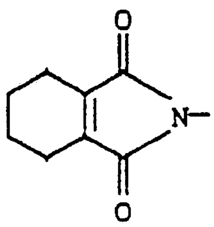 | F | $CH_3$ | $-COOCH_3$ | Fp 101 °C |
| 32 | 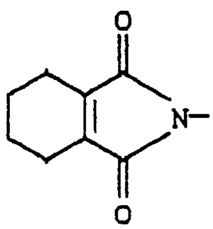 | F | $CH_3$ | $-(CH_2CH_2O)_2C_2H_5$ | Öl |
| 33 | 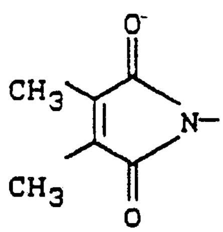 | F | $CH_3$ | $-(CH_2CH_2O)_2C_2H_5$ | Öl |
| 34 | 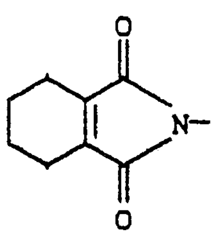 | F | $CH_3$ | $-CF_2-CHFCl$ | $^1$H-NMR*) 2.3(s); 6.17 (t); 6.41(t) |

Tabelle 1 (Fortsetzung)

| Bsp. Nr. | Het | $R^1$ | $R^2$ | $R^3$ | physikalische Eigenschaften |
|---|---|---|---|---|---|
| 35 | 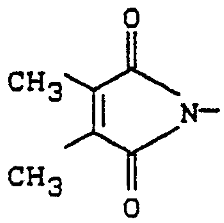 | F | $CH_3$ | $-CF_2-CHFCl$ | Fp 118 °C |
| 36 | 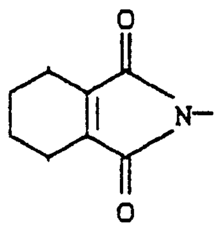 | F | $CH_3$ | 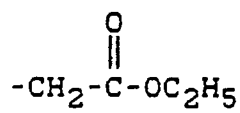 | Fp 106 °C |
| 37 | 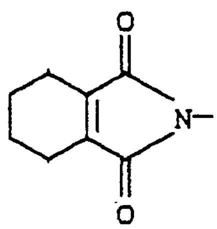 | F | $CH_3$ | 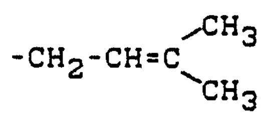 | Fp 102 °C |
| 38 | 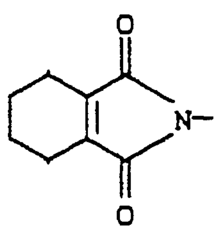 | F | $CH_3$ | 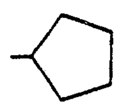 | Fp 84 °C |

<u>Tabelle 1</u> (Fortsetzung)

| Bsp. Nr. | Het | $R^1$ | $R^2$ | $R^3$ | physikalische Eigenschaften |
|---|---|---|---|---|---|
| 39 | 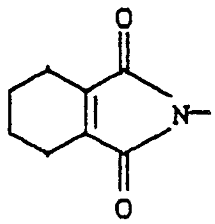 | F | $CH_3$ | 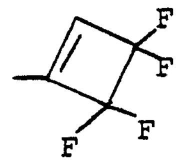 | Fp 134 °C |
| 40 | 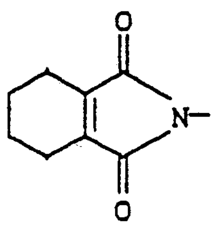 | F | $CH_3$ | $-CH_2-CH=CH-CH_3$ | Fp 155 °C |
| 41 | 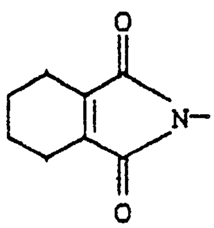 | F | $CH_3$ | 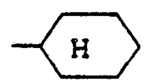 | Fp 86 °C |
| 42 | 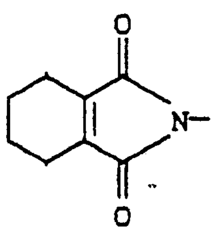 | F | $CH_3$ | 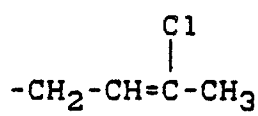 | Fp 139 °C |
| 43 | 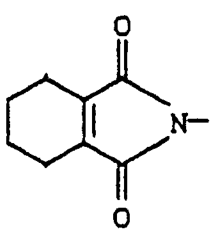 | F | $CH_3$ | 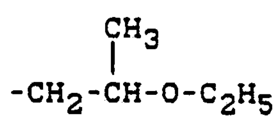 | Fp 92 °C |

Tabelle 1 (Fortsetzung)

| Bsp. Nr. | Het | $R^1$ | $R^2$ | $R^3$ | physikalische Eigenschaften |
|---|---|---|---|---|---|
| 44 | 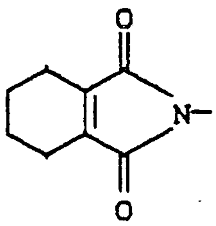 | F | $CH_3$ | 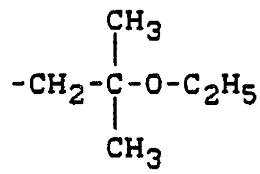 | $^1$H-NMR*): 2.25(s); 3.5 (q); 3.76 (s) |
| 45 | 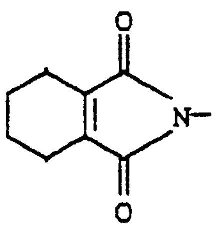 | F | $CH_3$ | 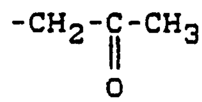 | Fp 121 °C |
| 46 | 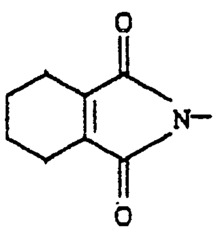 | F | $CH_3$ | 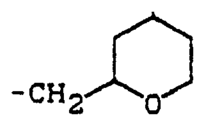 | Fp 95 °C |
| 47 | 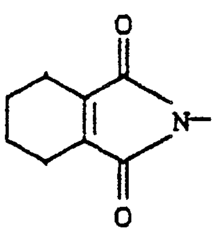 | F | $CH_3$ | 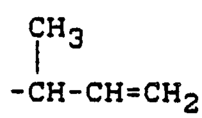 | $^1$H-NMR*): 4.67(m); 5.19(m); 5.89(dd) |
| 48 | 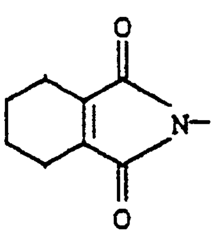 | F | $CH_3$ | 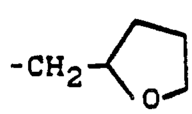 | Fp 88 °C |

Tabelle 1 (Fortsetzung)

| Bsp. Nr. | Het | $R^1$ | $R^2$ | $R^3$ | physikalische Eigenschaften |
|---|---|---|---|---|---|
| 49 | 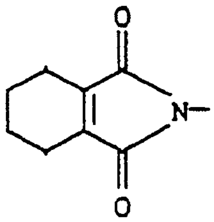 | F | $CH_3$ | 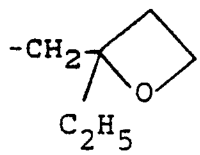 | Fp 91 °C |
| 50 | 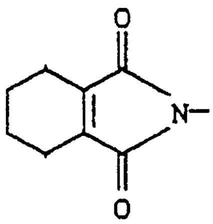 | F | $CH_3$ | 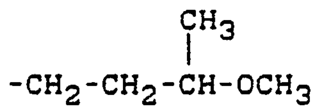 | Fp 70 °C |
| 51 | 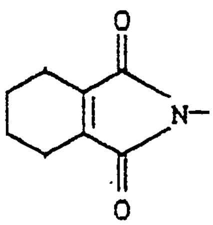 | F | $CH_3$ | 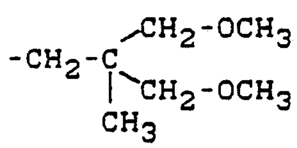 | $^1$H-NMR*): 1.05(s); 2.24(s); 3.31(s) 3.34(s); 3.77(s) |
| 52 | 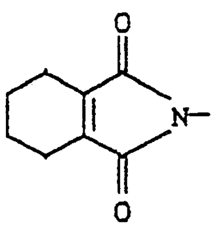 | F | $CH_3$ | 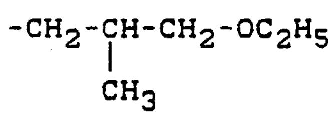 | $^1$H-NMR*): 1.05(d); 1.18(t); 2.22(s) 3.85(m) |
| 53 | 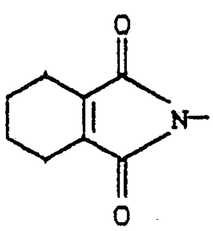 | F | $CH_3$ | 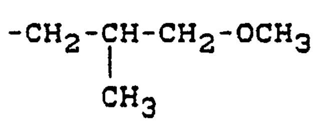 | $^1$H-NMR*): 1.05(d); 2.23(s) |

Tabelle 1 (Fortsetzung)

| Bsp. Nr. | Het | $R^1$ | $R^2$ | $R^3$ | physikalische Eigenschaften |
|---|---|---|---|---|---|
| 54 | 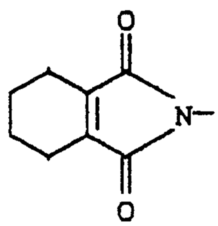 | F | $CH_3$ | $-CH_2-CH-CH_2-SCH_3$<br>$CH_3$ | $^1$H-NMR*):<br>1.13(d);<br>2.1(s);<br>3.85(m) |
| 55 | 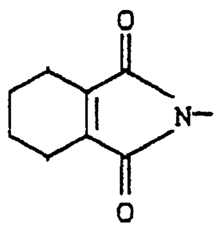 | F | $CH_3$ | 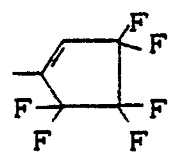 | Fp 150 °C |
| 56 | 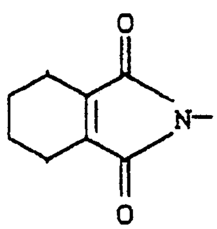 | F | $CH_3$ | $CH_3$<br>$-CH-CH_2-OC_2H_5$ | $^1$H-NMR*):<br>1.19(t);<br>1.3(d);<br>2.21(s);<br>4.39(m); |
| 57 | 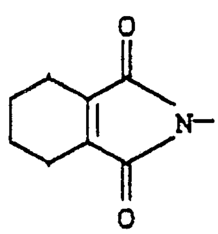 | F | $CH_3$ | $CH_3$<br>$-CH-C-CH_3$<br>O | Fp 135 °C |
| 58 | 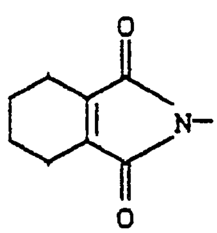 | F | $CH_3$ | $CH_3$<br>$-CH-CH_2-O-(CH_2)_2-CH_3$ | $^1$H-NMR*):<br>0.9(t);<br>1.28(d);<br>1.58(m);<br>4.4(m) |

Tabelle 1 (Fortsetzung)

| Bsp. Nr. | Het | $R^1$ | $R^2$ | $R^3$ | physikalische Eigenschaften |
|---|---|---|---|---|---|
| 59 | 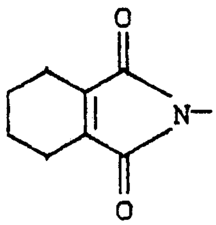 | F | $CH_3$ | $CH_3$<br>\|<br>$-CH-CH_2-O-CH(CH_3)_2$ | $^1H$-NMR*): 1.15(dd); 1.3(d); 4.35(m) |
| 60 | 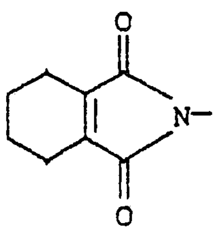 | F | $CH_3$ | $C_2H_5$<br>\|<br>$-CH-CH_2-O-C_2H_5$ | $^1H$-NMR*): 0.97(t); 1.17(t); 2.22(s); 4.2(m) |
| 61 | 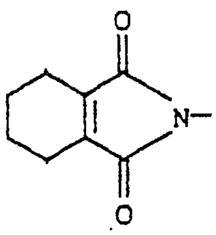 | F | $CH_3$ | $-CH_2-CH_2-OC_2H_5$ | Fp 50-59° C |
| 62 | 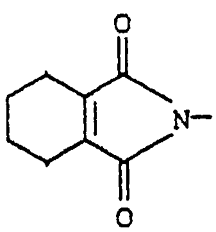 | F | $CH_3$ | $-CH_2-CH_2-O-CH_2-CF_3$ | Fp 80-85° C |

Tabelle 1 (Fortsetzung)

| Bsp. Nr. | Het | $R^1$ | $R^2$ | $R^3$ | physikalische Eigenschaften |
|---|---|---|---|---|---|
| 63 | 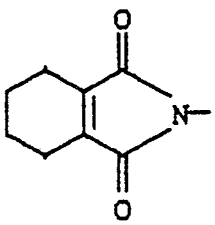 | F | $CH_3$ | $-CH_2-CH_2-O-CH_2-CF_3$ | Fp 83-86° C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen

Beispiel III-1

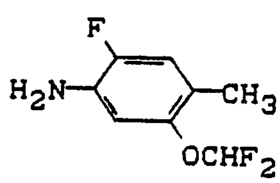

56 g (0,253 Mol) 4-Fluor-2-difluormethoxy-4-nitro-toluol und 10 g Platin auf Aktivkohle (1 % ig) in 400 ml Ethanol werden bei Raumtemperatur und Normaldruck bis zur Konstanz der Wasserstoffaufnahme hydriert. Zur Aufarbeitung wird der Katalysator abfiltriert und das Lösungsmittel im Vakuum entfernt.

Man erhält 42 g (86 % der Theorie) an 5-Difluormethoxy-2-fluor-4-methyl-anilin als Öl.

$^1$H-NMR (DMSO-$d_6$) : δ = 6,98 (t, 1H, $-OC\underline{H}F_2$) ppm.

Beispiel III-2

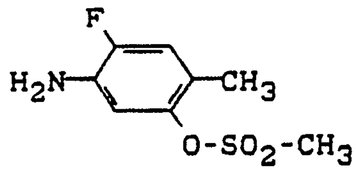

25 g (0,1 Mol) Methansulfonsäure-(4-fluor-2-methyl-5-nitrophenyl)-ester in 150 ml Ethoxyethanol werden mit 4 g Platin auf Aktivkohle (1 %ig) versetzt und bei Raumtemperatur und Normaldruck bis zur Konstanz der Wasserstoffaufnahme hydriert. Zur Aufarbeitung wird der Katalysator abfiltriert und das Lösungsmittel im Vakuum entfernt.

Man erhält 19,2 g (87,7 % der Theorie) an Methansulfonsäure-(4-fluor-2-methyl-5-amino-phenyl)-ester vom Schmelzpunkt 66 °C - 69 °C.

Beispiel V-1

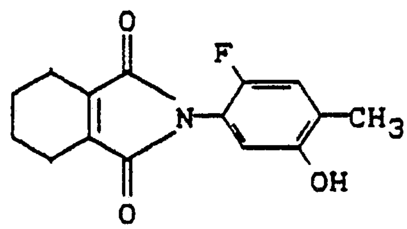

7,05 (0,05 Mol) 5-Amino-4-fluor-2-methyl-phenyl, 7,61 g (0,05 Mol) 3,4,5,6-Tetrahydrophthalsäureanhydrid und 0,1 g 4-(N,N-Dimethylamino)-pyridin in 80 ml Eisessig werden 2 Stunden auf Rückflußtemperatur erhitzt, abgekühlt und in 400 ml Eiswasser gegossen. Der so erhaltene Niederschlag wird abgesaugt, getrocknet und aus Chloroform/n-Hexan umkristallisiert.

Man erhält 9,97 g (72,5 % der Theorie) an N-(2-Fluor-5-hydroxy-4-methyl-phenyl)-3,4,5,6-tetrahydrophthalimid vom Schmelzpunkt 165 °C.

Beispiel V-2

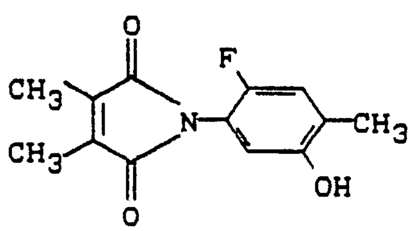

5,8 g (0,046 Mol) Dimethylmaleinsäureanhydrid, 6,49 g (0,046 Mol) 5-Amino-4-fluor-2-methylphenol und 0,1 g 4-(N,N-Dimethylamino)-pyridin in 70 ml Eisessig werden 2 Stunden auf Rückflußtemperatur erhitzt, abgekühlt und in 350 ml Eiswasser gegossen. Der so erhaltene Niederschlag wird abgesaugt und getrocknet.

Man erhält 8,34 g (73 % der Theorie) an N-(2-Fluor-5-hydroxy-4-methyl-phenyl)-3,4-dimethyl-$\Delta^3$-pyrrolin-2,5-dion vom Schmelzpunkt 145 °C.

Beispiel X-1

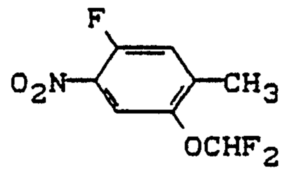

In eine Mischung aus 49 g (0,287 Mol) 4-Fluor-2-methyl-5-nitro-phenol und 45 ml Natronlauge (45 %ig) in 350 ml Dioxan wird bei 50 °C bis zur Sättigung Chlordifluormethan eingeleitet, anschließend eine Stunde bei Raumtemperatur gerührt, mit Eiswasser verdünnt und mit Ether extrahiert. Die vereinigten organischen Phasen werden mit Wasser gewaschen, über Natriumsulfat getrocknet und im Vakuum eingeengt.

Man erhält 58 g (91 % der Theorie) an 2-Difluormethoxy-5-fluor-4-nitro-toluol als Öl vom Brechungsindex $n_D^{20}$ = 1.5220.

Beispiel X-2

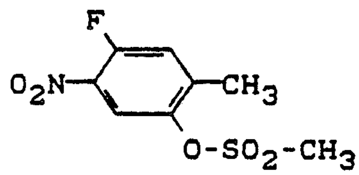

20 g (0,117 Mol) 4-Fluor-2-methyl-5-nitro-phenol in 40 ml Pyridin werden bei 10 °C unter Rühren tropfenweise mit 13,4 ml (0,117 Mol) Methansulfonsäurechlorid versetzt, anschließend im Vakuum eingeengt, der Rückstand in Dichlormethan aufgenommen und nacheinander mit verdünnter Salzsäure und Wasser gewaschen, über Magnesiumsulfat getrocknet und im Vakuum vom Lösungsmittel befreit.

Man erhält 26,5 g (91 % der Theorie) an Methansulfonsäure-(4-fluor-2-methyl-5-nitro-phenyl)ester vom Schmelzpunkt 96 °C - 97 °C.

Beispiel X-3

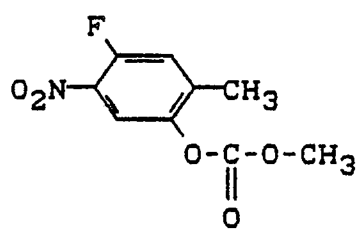

65 g (0,35 Mol) (2-Methyl-4-fluorphenyl)-methylcarbonat in 130 ml Dichlormethan werden bei 20 °C tropfenweise unter Rühren mit 65 g eines Gemisches aus konzentrierter Salpetersäure und konzentrierter Schwefelsäure (1:2) versetzt, anschließend 2 Stunden bei 20 °C und eine weitere Stunde bei 30 °C gerührt, abgekühlt, auf Eis gegossen, mit Dichlormethan extrahiert und destilliert.

Man erhält 62 g (76,7 % der Theorie) an 5-Fluor-2-methoxycarbonyloxy-4-nitro-toluol vom Siedepunkt 120 °C - 215 °C bei 0,27 mbar und vom Schmelzpunkt 68 °C.

Beispiel IX-1

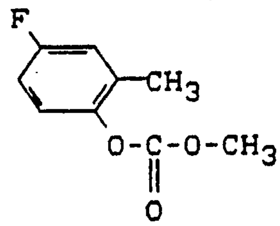

Zu 42 g (1,05 Mol) Natriumhydroxid in 660 ml Wasser gibt man 106 g (0,84 Mol) 2-Methyl-4-fluorphenol, tropft anschließend unter Rühren bei 5 °C 108 g (1,14 Mol) Chlorameisensäuremethylester zu, rührt nach beendeter Zugabe weitere 2 Stunden bei 5 °C, extrahiert mit Dichlormethan, trocknet über Natriumsulfat und destilliert im Vakuum.

Man erhält 139 g (88 % der Theorie) an Methyl-(4-fluor-2-methylphenyl)-carbonat vom Siedepunkt 98 °C bis 100 °C bei 16 mbar und vom Brechungsindex $n_D^{20}$ 1,4770.

Beispiel XII-1

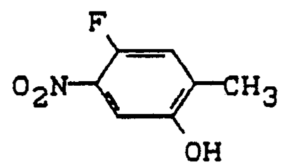

156 g (0,68 Mol) Methyl-(4-fluor-2-methyl-5-nitrophenyl)-carbonat werden in einer Mischung aus 400 ml Dioxan und 300 ml konzentrierter Salzsäure 12 Stunden auf Rückflußtemperatur erhitzt, anschließend abgekühlt, im Vakuum eingeengt und der Rückstand in Methyl-t-butylether aufgenommen. Man extrahiert mit 400 ml 10prozentiger Natronlauge, säuert die wässrige Phase an, filtriert den ausgefallenen Feststoff ab und kristallisiert aus Toluol um.

Man erhält 86 g (73,5 % der Theorie) an 4-Fluor-2-methyl-5-nitrophenol vom Schmelzpunkt 138 °C - 142 °C.

Beispiel XIII-1

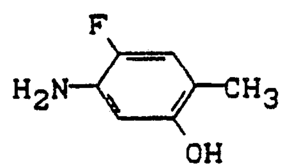

54 g (0,316 Mol) 4-Fluor-2-methyl-5-nitro-phenol in 800 ml Ethanol werden in Gegenwart von 3 g Platinoxid mit Wasserstoff bei 1 bis 5 bar und 20 °C bis 30 °C bis zur Konstanz der Wasserstoffaufnahme hydriert. Zur Aufarbeitung wird der Katalysator abfiltriert, das Lösungsmittel im Vakuum abdestilliert und der Rückstand aus Toluol umkristallisiert.

Man erhält 36,1 g (81 % der Theorie) an 5-Amino-4-fluor-2-methylphenol vom schmelzpunkt 145 °C.

Anwendungsbeispiele

In den folgenden Anwendungsbeispielen wurden die nachstehend aufgeführten Verbindungen als Vergleichsubstanzen eingesetzt:

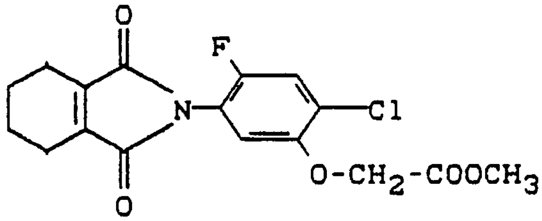

(A)

(bekannt aus EP-A 83 055 / Verbindung Nr. 1)

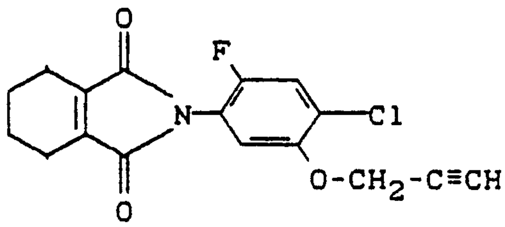

(B)

(bekannt aus EP-A 61 741 / Verbindung Nr. 10)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine Deutliche Überlegenheit in der Wirksamkeit ebenso wie in der Nutzpflanzenselektivität gegenüber dem Stand der Technik zeigt in diesem Test z.B. die Verbindungen gemäß Herstellungsbeispiel 7.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 5, 7 und 8.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes austrocknen der Blätter, geringer Blattfall
\+ + starkes Austrocknen der Blätter, starker Blattfall
\+ + + sehr starkes Austrocknen der Blätter, sehr starker Blattfall
Eine deutliche Überlegenheit im Vergleich zur unbehandelten Kontrolle zeigen in diesem Test beispielsweise die Verbindungen gemäß folgender Herstellungsbeispiele: 4, 5, 7 und 8.

**Ansprüche**

1. N-Aryl-Stickstoffheterocyclen der allgemeinen Formel (I),

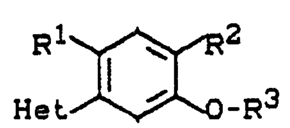

(I)

in welcher

Het für einen Heterocyclus der Formel

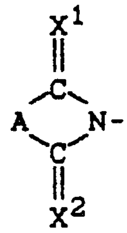 oder 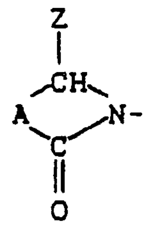 steht,

$R^1$ für Wasserstoff oder Halogen steht,
$R^2$ für Alkyl steht und
$R^3$ für jeweils gegebenenfalls substituiertes Alkyl, Alkenyl, Alkinyl, Cycloalkyl oder Cycloalkenyl steht oder für
einen Rest $-\underset{\underset{O}{\|}}{C}-R^4$
oder einen Rest $-SO_2-R^5$ steht,
A für einen Rest der Formel

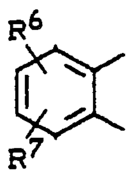 ; 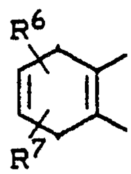 ; 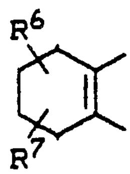 ; 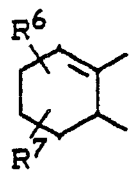 ;

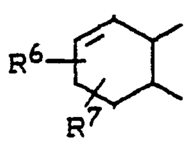 ; 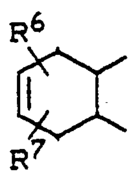 oder 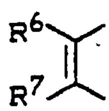 steht,

$X^1$ und $X^2$ jeweils für Sauerstoff oder Schwefel stehen,
Z für Wasserstoff, Hydroxy oder Chlor steht,
$R^4$ für Alkyl, Alkoxy oder Halogenalkyl steht,
$R^5$ für Alkyl, Halogenalkyl oder für gegebenenfalls substituiertes Aryl steht und
$R^6$ und $R^7$ unabhängig voneinander jeweils für Wasserstoff, Halogen, Alkyl oder Halogenalkyl stehen,
wobei jedoch die Verbindung N-[3-(1-Cyanoethoxy)-4-methyl-phenyl]-3,4,5,6-tetrahydrophthalimid ausgenommen ist.

2. N-Aryl-Stickstoffheterocyclen der Formel (I) gemäß Anspruch 1, in welcher
Het für einen Heterocyclus der Formel

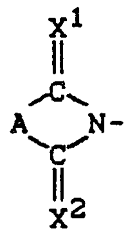 oder 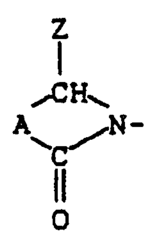 steht,

$R^1$ für Wasserstoff, Fluor, Chlor oder Brom steht,
$R^2$ für geradkettiges oder verzweigtes Alkyl mit 1 bis 4 Kohlenstoffatomen steht,
$R^3$ für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1

bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen steht, außerdem für jeweils geradkettiges oder verzweigtes Cyanalkyl, Alkoxyalkyl, Alkylthioalkyl, Halogenalkoxyalkyl, Alkoxyalkoxyalkyl, Alkoxyalkoxyalkoxyalkyl, Bis(alkoxy)alkyl, Bis(alkylthio)alkyl, Alkyl carbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen steht, ferner für jeweils gegebenenfalls einfach oder mehrfach, gleich oder verschieden substituiertes Cycloalkyl, Cycloalkenyl, Cycloalkyloxycarbonylalkyl oder Cycloalkylalkyl mit jeweils 3 bis 7 Kohlenstoffatomen in Cycloalkylteil bzw. Cycloalkenylteil und gegebenenfalls 1 bis 4 Kohlenstoffatomen im geradkettigen oder verzweigten Alkylteil steht, wobei als Substituenten jeweils infrage kommen: Halogen sowie jeweils geradkettiges oder verzweigtes Alkyl oder Alkoxy mit jeweils 1 bis 4 Kohlenstoffatomen, $R^3$ außerdem für jeweils gegebenenfalls einfach oder mehrfach, gleich oder verschieden substituiertes Oxiranylalkyl, Oxetanylalkyl, Tetrahydrofuranylalkyl oder Tetrahydropyranylalkyl mit jeweils 1 bis 4 Kohlenstoffatomen im geradkettigen oder verzweigten Alkylteil steht, wobei als Substituenten jeweils infrage kommen: Halogen sowie jeweils geradkettiges oder verzweigtes Alkyl oder Alkoxy mit jeweils 1 bis 4 Kohlenstoffatomen; $R^3$ weiterhin für gegebenenfalls einfach oder mehrfach gleich oder verschieden substituiertes Aralkyl mit 6 bis 10 Kohlenstoffatomen im Arylteil und 1 bis 4 Kohlenstoffatom im geradkettigen oder verzweigten Alkylteil steht, wobei als Substituenten infrage kommen:

Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen; oder $R^3$ ferner für einen Rest $-\underset{\underset{O}{\|}}{C}-R^4$

oder für einen Rest $-SO_2-R^5$ steht,

A für einen Rest der Formel

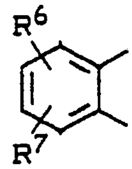 ; 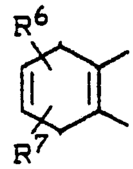 ; 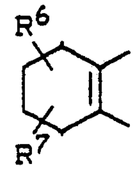 ; 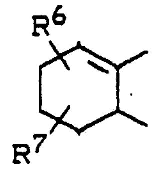 ;

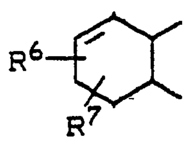 ; 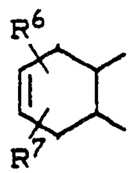 oder 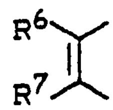 steht,

$X^1$ und $X^2$ jeweils für Sauerstoff oder Schwefel stehen,

Z für Wasserstoff, Hydroxy oder Chlor steht,

$R^4$ für jeweils geradkettiges oder verzweigtes Alkyl, Alkoxy oder Halogenalkyl mit jeweils 1 bis 6 Kohlenstoffatomen und im Fall des Halogenalkyl mit 1 bis 13 gleichen oder verschiedenen Halogenatomen steht,

$R^5$ für jeweils geradkettiges oder verzweigtes Alkyl oder Halogenalkyl mit jeweils 1 bis 6 Kohlenstoffatomen und im Fall des Halogenalkyl mit 1 bis 13 gleichen oder verschiedenen Halogenatomen steht und außerdem für gegebenenfalls einfach oder mehrfach, gleich oder verschieden substituiertes Phenyl steht, wobei als Substituenten infrage kommen:

Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen und

$R^6$ und $R^7$ unabhängig voneinander jeweils für Wasserstoff, Fluor, Chlor, Brom oder für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen, stehen,

wobei jedoch die Verbindung N-[3-(1-Cyanoethoxy)-4-methyl-phenyl]-3,4,5,6-tetrahydrophthalimid ausgenommen ist.

3. Verfahren zur Herstellung von N-Aryl-Stickstoffheterocyclen der allgemeinen Formel (I),

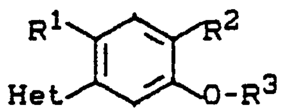

(I)

in welcher
Het für einen Heterocyclus der Formel

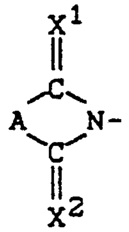

oder

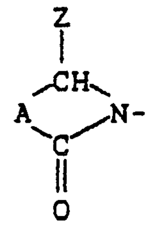

steht,

$R^1$ für Wasserstoff oder Halogen steht,
$R^2$ für Alkyl steht,
$R^3$ für jeweils gegebenenfalls substituiertes Alkyl, Alkenyl, Alkinyl, Cycloalkyl oder Cycloalkenyl steht oder für
einen Rest $-\underset{\underset{O}{\|}}{C}-R^4$
oder einen Rest $-SO_2-R^5$ steht, wobei
A für einen Rest der Formel

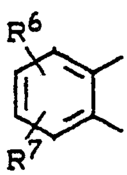

;

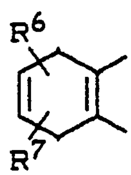

;

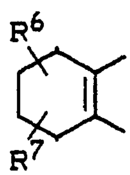

;

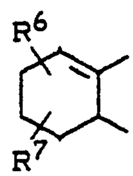

;

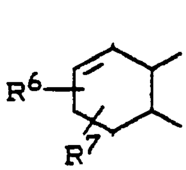

;

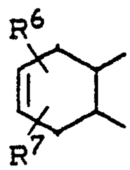

oder

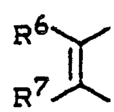

steht,

$X^1$ und $X^2$ jeweils für Sauerstoff oder Schwefel stehen,
Z für Wasserstoff, Hydroxy oder Chlor steht,
$R^4$ für Alkyl, Alkoxy oder Halogenalkyl steht,
$R^5$ für Alkyl, Halogenalkyl oder für gegebenenfalls substituiertes Aryl steht und
$R^6$ und $R^7$ unabhängig voneinander jeweils für Wasserstoff, Halogen, Alkyl oder Halogenalkyl stehen,
wobei jedoch die Verbindung N-[3-(1-Cyanoethoxy)-4-methyl-phenyl]-3,4,5,6-tetrahydrophthalimid ausgenommen ist,
dadurch gekennzeichnet, daß man
(a) N-Arylstickstoffheterocyclen der Formel (Ia),

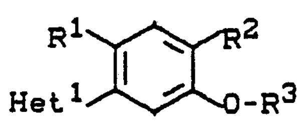

(Ia)

in welcher
Het¹für einen Rest der Formel

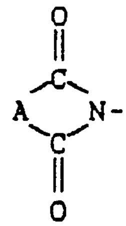 steht und

$R^1$, $R^2$, $R^3$ und Adie oben angegebene Bedeutung haben, erhält, wenn man Anhydride der Formel (II),

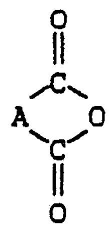

(II)

in welcher
A die oben angegebene Bedeutung hat,
mit Aminen der Formel (III),

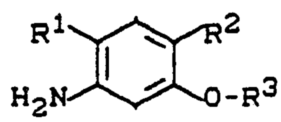

(III)

in welcher
$R^1$, $R^2$ und $R^3$ die oben angegebene Bedeutung haben,
gegebenenfalls in Gegenwart eines Verdünnungsmittels und gegebenenfalls in Gegenwart eines Reaktionshilfsmittels umsetzt; oder daß man

(b) N-Aryl-Stickstoffheterocyclen der Formel (Ib),

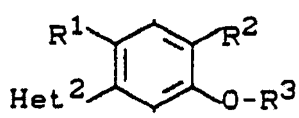

(Ib)

in welcher
$Het^2$ für einen Rest der Formel

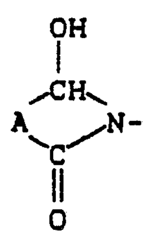 steht und

$R^1$, $R^2$, $R^3$ und A die oben angegebene Bedeutung haben, erhält, wenn man N-Arylstickstoffheterocyclen der Formel (Ia),

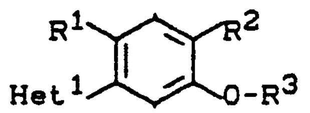

(Ia)

in welcher
Het¹ für einen Rest der Formel

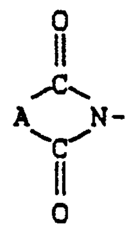

steht und

A, $R^1$, $R^2$ und $R^3$ die oben angegebene Bedeutung haben, mit einem Reduktionsmittel, gegebenenfalls in Gegenwart eines Verdünnungsmittels umsetzt; oder daß man

(c) N-Aryl-Stickstoffheterocyclen der Formel (Ic),

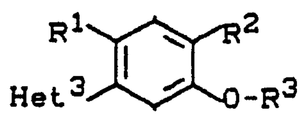

(Ic)

in welcher
Het³ für einen Rest der Formel

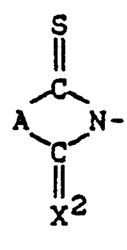

steht und

$X^2$, A, $R^1$, $R^2$ und $R^3$ die oben angegebene Bedeutung haben, erhält, wenn man N-Aryl-Stickstoffheterocyclen der Formel (Ia),

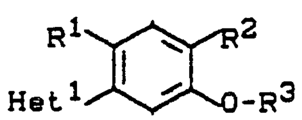

(Ia)

Het¹ für einen Rest der Formel

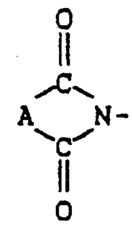 steht und

A, $R^1$, $R^2$ und $R^3$ die oben angegebene Bedeutung haben, mit "Lawessons-Reagenz" der Formel (IV),

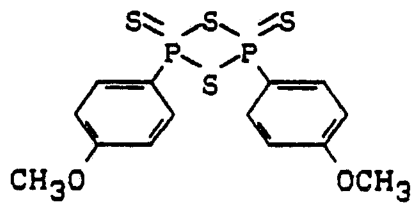

(IV)

gegebenenfalls in Gegenwart eines Verdünnungsmittels umsetzt; oder daß man

(d) N-Aryl-Stickstoffheterocyclen der Formel (Id),

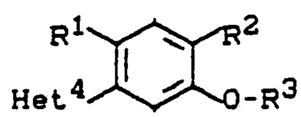

(Id)

in welcher

$Het^4$ für einen Rest der Formel

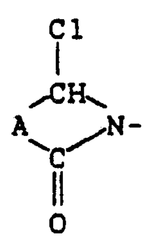 steht und

A, $R^1$, $R^2$ und $R^3$ die oben angegebene Bedeutung haben, erhält, wenn man N-Aryl-Stickstoffheterocyclen der Formel (Ib),

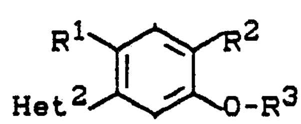

(Ib)

in welcher

$Het^2$ für einen Rest der Formel

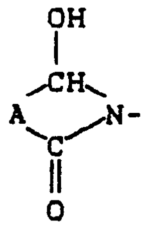 steht und

A, $R^1$, $R^2$ und $R^3$ die oben angegebene Bedeutung haben, mit Thionylchlorid gegebenenfalls in Gegenwart eines Verdünnungsmittels und gegebenenfalls in Gegenwart eines Reaktionshilfsmittels umsetzt; oder daß man

(e) N-Aryl-Stickstoffheterocyclen der Formel (Ie),

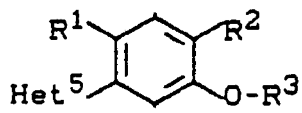

(Ie)

in welcher

$Het^5$ für einen Rest der Formel

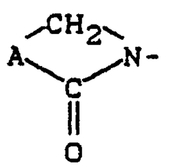 steht und

A, $R^1$, $R^2$ und $R^3$ die oben angegebene Bedeutung haben, erhält, wenn man N-Aryl-Stickstoffheterocyclen der Formel (Id),

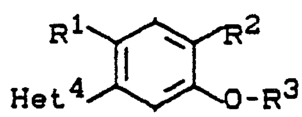

(Id)

in welcher

$Het^4$ für einen Rest der Formel

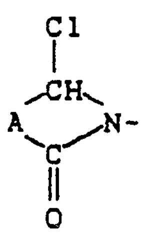 steht und

A, $R^1$, $R^2$ und $R^3$ die oben angegebene Bedeutung haben, mit molekularem Wasserstoff in Gegenwart eines Katalysators sowie gegebenenfalls in Gegenwart eines Verdünnungsmittels und gegebenenfalls in Gegenwart eines Säurebindemittels umsetzt; oder daß man

(f) N-Aryl-Stickstoffheterocyclen der Formel (Ia),

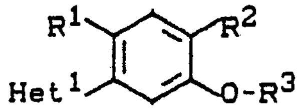

(Ia)

in welcher
Het[1] für einen Rest der Formel

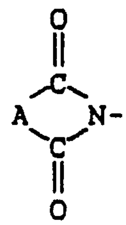 steht und

A, $R^1$, $R^2$ und $R^3$ die oben angegebene Bedeutung haben, erhält, wenn man Phenole der Formel (V),

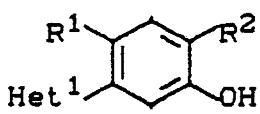

(V)

in welcher
Het[1], $R^1$ und $R^2$ die oben angegebene Bedeutung haben,
($\alpha$) mit Alkylierungsmitteln der Formel (VI),

$R^{3-1}$-$E^1$ (VI)

in welcher
$R^{3-1}$ für jeweils gegebenenfalls substituiertes Alkyl, Alkenyl, Alkinyl, Cycloalkyl oder Cycloalkenyl steht und
$E^1$ für eine elektronenanziehende Abgangsgruppe steht, oder
($\beta$) mit Acylierungsmitteln der Formel (VII),

$R^4-\underset{\overset{\|}{O}}{C}-E^2$ (VII)

in welcher
$R^4$ die oben angegebene Bedeutung hat und
$E^2$ für eine elektronenanziehende Abgangsgruppe steht, oder
($\gamma$) mit Sulfonylierungsmitteln der Formel (VIII),

$R^5$-$SO_2$-$E^3$ (VIII)

in welcher
$R^5$ die oben angegebene Bedeutung hat und
$E^3$ für eine elektronenanziehende Abgangsgruppe steht,
jeweils gegebenenfalls in Gegenwart eines Verdünnungsmittels und gegebenenfalls in Gegenwart eines Reaktionshilfsmittels umsetzt.

4. Herbizide und pflanzenwuchsregulierende Mittel, gekennzeichnet durch einen Gehalt an mindestens einem N-Aryl-Stickstoffheterocyclus der Formel (I) gemäß den Ansprüchen 1 bis 3.

5. Verfahren zur Bekämpfung von Unkräutern, dadurch gekennzeichnet, daß man N-Aryl-Stickstoffheterocyclen der Formel (I) gemäß den Ansprüchen 1 bis 3 auf die Unkräuter und/oder ihren Lebensraum einwirken läßt.

6. Verwendung von N-Aryl-Stickstoffheterocyclen der Formel (I) gemäß den ansprüchen 1 bis 3 zur Bekämpfung von Unkräutern und/oder als Pflanzenwachstumsregulatoren.

7. Verfahren zur Herstellung von herbiziden und/oder pflanzenwuchsregulierenden Mitteln, dadurch gekennzeichnet, daß man N-Aryl-Stickstoffheterocyclen der Formel (I) gemäß den Ansprüchen 1 bis 3 mit Streckmitteln und/oder oberflächenaktiven Substanzen vermischt.

8. Amine der Formel (IIIa)

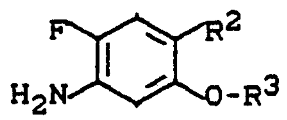

(IIIa)

in welcher
$R^2$ für Alkyl steht und
$R^3$ für jeweils gegebenenfalls substituiertes Alkyl, Alkenyl, Alkinyl, Cycloalkyl oder Cycloalkenyl steht oder für einen Rest $-\underset{\underset{O}{\|}}{C}-R^4$
oder einen Rest $-SO_2-R^5$ steht, wobei,
$R^4$ für Alkyl, Alkoxy oder Halogenalkyl steht und
$R^5$ für Alkyl, Halogenalkyl oder für gegebenenfalls substituiertes Aryl steht.

9. Phenole der Formel (Va)

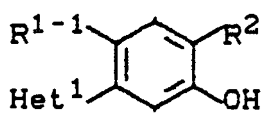

(Va)

in welcher
$R^{1-1}$ für Halogen steht,
$R^2$ für Alkyl steht und
$Het^1$ für einen Rest der Formel

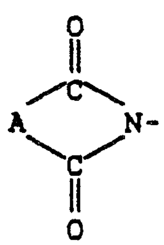

steht, wobei

A für einen Rest der Formel

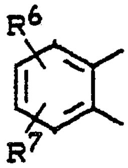 ; 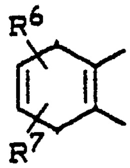 ; 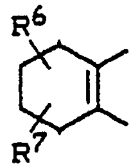 ; 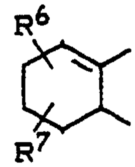 ;

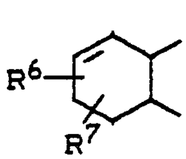 ; 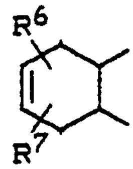 oder 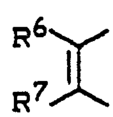 steht und

$R^6$ und $R^7$ unabhängig voneinander jeweils für Wasserstoff, Halogen, Aklyl oder Halogenalkyl stehen.

10. Nitroverbindungen der Formel (X)

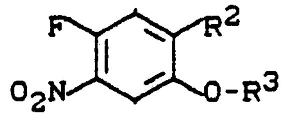

(X)

in welcher

$R^2$ für Alkyl steht und

$R^3$ für jeweils gegebenenfalls substituiertes Alkyl, Alkenyl, Alkinyl, Cycloalkyl oder Cycloalkenyl steht oder für einen Rest $-\underset{\underset{O}{\|}}{C}-R^4$

oder einen Rest $-SO_2-R^5$ steht,

wobei

$R^4$ für Alkyl, Alkoxy oder Halogenalkyl steht und

$R^5$ für Alkyl, Halogenalkyl oder für gegebenenfalls substituiertes Aryl steht.